# EUROPEAN PATENT APPLICATION

(11) **EP 3 936 519 A1**
(43) Date of publication of application: **12.01.2022**
(21) Application number: 20767245.2
(22) Date of filing: 24.02.2020
(51) Int. Cl.: C07K 14/415, C12N 15/29, C12N 15/82, A01H 5/00, A01H 6/46

(54) **METHOD FOR CULTIVATING PLANT RESISTANT TO GRAY LEAF SPOT**

(30) Priority: 04.03.2019 CN 201910160206
(71) Applicant: China Agricultural University, Beijing 100193 (CN)
(72) Inventor: XU, Ming Liang, Beijing 100193 (CN); ZHU, Mang, Beijing 100193 (CN); FAN, Xing Ming, Beijing 100193 (CN); ZHONG, Tao, Beijing 100193 (CN); XU, Ling, Beijing 100193 (CN); ZHANG, Yan, Beijing 100193 (CN); LIU, Li, Beijing 100193 (CN)
(74) Representative: f & e patent
(86) International application number: PCT/CN2020/076319
(87) International publication number: WO 2020/177560

(57) **Abstract**

The present invention discloses a method for cultivating a plant resistant to gray leaf spot. The proteins provided by the present invention are obtained from corn and named as ZMPK protein, and are the proteins represented by seq. 2, seq. 4, seq. 7 or seq. 9 in the sequence list. Nucleic acid molecules encoding the ZMPK proteins are also within the scope of the present invention. The invention further sets forth a method for preparing a transgenic plant, comprising the step of: introducing the nucleic acid molecules into a starting plant to obtain the transgenic plant with reduced resistance to gray leaf spot. The invention further sets forth a method for preparing a transgenic plant, comprising the step of: knocking out or inhibiting the expression of the nucleic acid molecules in a starting plant to obtain the transgenic plant with increased resistance to gray leaf spot. The present invention is of great application value to the breeding of corn resistant to gray leaf spot.

## Description

### Technical Field

The present invention belongs to the field of biotechnology, and specifically relates to a method for cultivating plants resistant to gray leaf spot; more specifically, it relates to a method for genetically improving plants by expressing a *ZmPK* gene to obtain plants resistant to gray leaf spot.

### Background Art

Corn gray leaf spot is a worldwide fungal disease of corn leaves. It was first discovered in Alexandria County, Illinois, USA in 1925, and seriously affected the yield of corn. In China, the corn gray leaf spot first occurred in Dandong City, Liaoning Province in 1991. After that, it was reported in Jilin, Hebei, Yunnan and other regions. In recent years, gray leaf spot has become one of the main leaf diseases in corn production in China, especially in the southwest corn producing areas. The occurrence of corn gray leaf spot generally causes a 10-30% reduction in corn production, and in severe cases, it can reach 60-80% or even no harvest, which severely affects the production of corn in China.

Current researchers believe that there are two main pathogens causing corn gray leaf spot: *Cercospora zeae-maydis* (*Czm*) and *Cercospora zeina* (*Cz*)*.* For a long time, domestic researchers believed that the pathogen of corn gray leaf spot in China was *Czm.* Liu et al. took samples in the Yunnan area and analyzed the morphology, pathogenicity, ITS sequence and histone H3 gene sequence of the microorganism and found that the pathogen of corn gray leaf spot in the Yunnan area in China is *Cz.* The disease spots first appeared on the lower leaves, and the symptoms were most obvious on the leaves. In the early stage of onset, the disease spots were water-stained faded-green spots, and then expanded to grayish-brown color spots, which were approximately rectangular and parallel to the veins of the leaves. When the disease is severe, the disease spots expand and spread, causing the leaves to wither. The use of fungicides and other chemical control methods is not effective. Breeding varieties resistant to gray leaf spot is the most economical and effective way to control this disease.

### Summary of the Invention

The present invention provides a method for cultivating plants resistant to gray leaf spot.

The present invention provides a protein, which is obtained from corn, and named as ZmPK protein, which is as follows: (a1), or (a2), or (a3), or (a4), or (a5), or (a6), or (a7), or (a8):
(a1) a protein represented by SEQ ID NO: 2 in the sequence listing;
(a2) a protein represented by SEQ ID NO: 4 in the sequence listing;
(a3) a protein represented by SEQ ID NO: 7 in the sequence listing;
(a4) a protein represented by SEQ ID NO: 9 in the sequence listing;
(a5) a fusion protein obtained by attaching a tag to an N-terminus or/and a C-terminus of the protein in any one of (a1) to (a4);
(a6) a protein comprising the following three segments from N-terminus to C-terminus: the protein in any one of (a1) to (a4), a connecting peptide, and an EGFP protein;
(a7) a protein related to plant gray leaf spot resistance obtained by substituting and/or deleting and/or adding one or a plurality of amino acid residues to the protein in any one of (a1) to (a6); and
(a8) a protein related to plant gray leaf spot resistance obtained from corn and having a homology of 90%, 91%, 92%, 93%, 94%, 95%, 96%, 97%, 98% or 99% with the protein in any one of (a1) to (a4).

The tags are as shown in Table 1 below.

**Table 1 Tag sequences**

| Tag | Residues | Sequence |
|---|---|---|
| Poly-Arg | 5 to 6 (typically 5) | RRRR |
| Poly-His | 2 to 10 (typically 6) | HHHHHH |
| FLAG | 8 | DYKDDDDK |
| Strep-tag II | 8 | WSHPQFEK |
| c-myc | 10 | EQKLISEEDL |
| HA | 9 | YPYDVPDYA |
| EGFP | 239 | Sequence 15 |

The EGFP protein is specifically shown in Sequence 15 in the sequence Listing. The connecting peptide can be specifically as shown in Sequence 19 in the sequence listing.

The protein can be synthesized artificially, or its encoding gene can be synthesized first, and then the protein can be obtained by biological expression.

The nucleic acid molecule encoding the ZmPK protein also falls within the scope of protection of the present invention.

The nucleic acid molecule is any one of the following (b1) to (b15):
(b1) a DNA molecule with an encoding region that is represented by nucleotides 56 to 1618 in SEQ ID NO: 3 in the sequence listing;
(b2) a DNA molecule represented by SEQ ID NO: 3 in the sequence listing;
(b3) a DNA molecule with an encoding region that is represented by nucleotides 56 to 1624 in SEQ ID NO: 5 in the sequence listing;
(b4) a DNA molecule represented by SEQ ID NO: 5 in the sequence listing;
(b5) a DNA molecule represented by SEQ ID NO: 1 in the sequence listing;
(b6) a DNA molecule with an encoding region that is represented by nucleotides 56 to 1618 in SEQ ID NO: 8 in the sequence listing;
(b7) a DNA molecule represented by SEQ ID NO: 8 in the sequence listing;
(b8) a DNA molecule with an encoding region that is represented by nucleotides 56 to 1624 in SEQ ID NO: 10 in the sequence listing;
(b9) a DNA molecule represented by SEQ ID NO: 10 in the sequence listing;
(b10) a DNA molecule represented by SEQ ID NO: 6 in the sequence listing;
(b11) a DNA molecule represented by SEQ ID NO: 12 in the sequence listing;
(b12) a DNA molecule represented by SEQ ID NO: 13 in the sequence listing;
(b13) a DNA molecule represented by SEQ ID NO: 14 in the sequence listing;
(b14) a DNA molecule that is derived from corn, has a homology of 90%, 91%, 92%, 93%, 94%, 95%, 96%, 97%, 98% or 99% with any one of (b1) to (b13), and encodes the protein;
(b15) a DNA molecule that hybridizes to any one of (b1) to (b13) under a stringent condition, and encodes the protein.

The stringent condition mentioned above is as follows: in a solution of 2×SSC, 0.1% SDS, hybridizing is performed at 68°C, and the membrane is washed twice for 5 min each time, and then hybridizing is performed again in a solution of 0.5×SSC, 0.1% SDS at 68°C, and the membrane is then washed twice for 15 min each time.

An expression cassette, recombinant vector or recombinant microorganism containing the nucleic acid molecule also falls within the scope of protection of the present invention.

The existing expression vectors can be used to construct a recombinant expression vector containing the nucleic acid molecule. When using the nucleic acid molecule to construct a recombinant expression vector, any enhanced, constitutive, tissue-specific or inducible promoter can be added before its transcription initiation nucleotide, and they can be used alone or in combination with other plant promoters that can be used in combination. In addition, when using the nucleic acid molecule to construct a recombinant expression vector, enhancers, including translation enhancers or transcription enhancers, can also be used. These enhancer regions can be ATG start codons or adjacent region start codons, etc., but they must be in the same reading frame with the coding sequence in order to ensure correct translation of the entire sequence. The sources of the translation control signals and initiation codons are extensive, and they can be natural or synthetic. The translation initiation region can be derived from a transcription initiation region or a structural gene. In order to facilitate the identification and screening of transgenic plants or transgenic microorganisms, the expression vectors used herein can be processed. For example, gene expressing enzymes or luminescent compounds that can produce color changes in plants or microorganisms, resistant antibiotic markers or chemical reagent resistant marker genes, etc. can be added herein. Considering the safety of the transgenes, it is possible to directly screen transformed plants or microorganisms by phenotype without adding any selectable marker genes.

The recombinant expression vector may specifically be a recombinant plasmid obtained by inserting the double-stranded DNA molecule shown in Sequence 12 in the sequence listing into the multiple cloning site (for example, the *Bam*HI site) of the pCAMBIA3301 vector.

The recombinant expression vector may specifically be a recombinant plasmid obtained by inserting the nucleic acid molecule into the multiple cloning site (for example, the XcmI restriction site) of the pBCXUN vector.

The present invention further sets forth the application of the ZmPK protein, which is the following (c1) or (c2): (c1) to regulate the resistance of a plant to gray leaf spot; and (c2) to reduce the disease resistance of a plant to gray leaf spot.

The present invention further sets forth the application of the nucleic acid molecule, which is the following (d1) or (d2): (d1) to cultivate a transgenic plant with altered resistance to gray leaf spot; and (d2) to cultivate a transgenic plant with reduced resistance to gray leaf spot.

The application of the nucleic acid molecule further includes using the nucleic acid molecule as a target to reduce the expression amount of the nucleic acid molecule. The implementation methods include, but are not limited to: RNAi interference, gene knockout, etc. The implementation methods also include: insertion, deletion or editing of the promoter region, and promoter interchange. The methods for the target may also include, but are not limited to: using editing or mutant alleles with lower expression or weaker activity, etc.

The present invention further sets forth an application of a substance for inhibiting an activity of the ZmPK protein in a plant and/or for reducing an abundance of the ZmPK protein in a plant to enhance the disease resistance of the plant to gray leaf spot.

The present invention further sets forth an application of a substance for inhibiting a transcription of the nucleic acid molecule and/or for inhibiting an expression of the nucleic acid molecule and/or for gene editing of the nucleic acid molecule to enhance the disease resistance of the plant to gray leaf spot. The "substance for gene editing of the nucleic acid molecule" may specifically be any interference vector described later or any gene editing vector described later.

The present invention further sets forth a method for preparing a transgenic plant, comprising the steps of: introducing the nucleic acid molecule into a starting plant to obtain a transgenic plant with reduced gray leaf spot resistance. The nucleic acid molecule can be specifically introduced into the starting plant through any one of the above-mentioned recombinant expression vectors. The recombinant expression vector carrying the nucleic acid molecule can be transformed into the starting plant by conventional biological methods such as Ti plasmid, Ri plasmid, plant virus vector, direct DNA transformation, microinjection, electrical conduction, and agrobacterium mediation. By crossing the transgenic plants with existing corn varieties (including single crosses and multiple crosses, such as three consecutive crosses), the obtained transgenic progeny plants are also transgenic plants with reduced gray leaf spot resistance. The existing corn variety may specifically be a corn inbred line Q11.

The present invention further sets forth a plant breeding method, comprising the following steps: increasing a content and/or activity of the ZmPK protein in a target plant, thereby reducing the gray leaf spot resistance of the target plant.

The present invention further sets forth a plant breeding method, comprising the following steps: inhibiting an expression of the nucleic acid molecule in a starting plant to obtain a transgenic plant with increased gray leaf spot resistance. Inhibiting the expression of the nucleic acid molecule in the starting plant can be specifically achieved by means of introducing an interference vector. The interference vector may specifically be the following recombinant plasmid: a recombinant plasmid having a forward fragment, a spacer fragment and a reverse fragment; the spacer segment is used to space the forward segment and the reverse segment; the forward segment and the reverse segment are in a reverse complementary relationship; the forward fragment is shown in Sequence 11 in the sequence listing. The interference vector may specifically be the following recombinant plasmid: a recombinant plasmid obtained by using the pGreen-HY104 vector as a starting vector, and inserting forward fragments and reverse fragments into different multiple cloning sites; the forward segment and the reverse segment are in a reverse complementary relationship; and the forward fragment is shown in Sequence 11 in the sequence listing. The interference vector may specifically be the following recombinant plasmid: an RNAi interference vector obtained by using the pGreen-HY104 vector as the starting vector, inserting a forward fragment between the *BamH*I and *Xba*I restriction sites, and inserting a reverse fragment between the *Hind*III and *EcoR*I restriction sites; the forward fragment and the reverse fragment are in a reverse complementary relationship; and the forward fragment is shown in Sequence 11 in the sequence listing. The interference vector can be transformed into the starting plant by conventional biological methods such as Ti plasmid, Ri plasmid, plant virus vector, direct DNA transformation, microinjection, electric conduction, agrobacterium mediation and the like. By crossing the transgenic plants with existing corn varieties (including single crosses and multiple crosses, such as three consecutive crosses), the obtained transgenic progeny plants are also transgenic plants with reduced gray leaf spot resistance. The existing corn variety may specifically be a corn inbred line Q11.

The present invention further sets forth a plant breeding method, comprising the following steps: reducing a content and/or activity of the protein ZmPK in a target plant, thereby increasing the disease resistance of the target plant to gray leaf spot.

The present invention further sets forth a plant breeding method, comprising the steps of: performing gene editing (causing a frameshift mutation in the specific gene) on a specific gene in a genome of a starting plant to increase the gray leaf spot resistance of the target plant; and the specific gene encoding the ZmPK protein.

The gene editing is specifically realized by the Cas9 technology.

The gene editing is specifically realized by two sgRNAs and the Cas9 protein, in which the target sequence binding region of one sgRNA is shown in Sequence 17 of the sequence listing, and the target sequence binding region of the other sgRNA is shown in Sequence 18 of the sequence listing.

The gene editing is specifically realized by introducing a gene editing vector. The gene editing vector may specifically be the following recombinant plasmid: a recombinant plasmid obtained by inserting the double-stranded DNA molecule shown in Sequence 16 in the sequence listing into the *Bsa*I restriction site of the pBUE411 vector.

Any of the above-mentioned plants is a dicotyledonous plant or a monocotyledonous plant. The monocotyledonous plant may be a gramineous plant. The gramineous plant may be a plant of the genus Zea. The Zea plant may specifically be corn, such as corn inbred line B73 or corn inbred line B73-329.

### Any of the above gray leaf spots may specifically be gray leaf spot caused by Cercospora cornae.

### Brief Description of the Drawings

Fig. 1 is a schematic diagram of the structure of a pBCXUN vector.
Fig. 2 is the results of the identification of gene expression level in Example 2.
Fig. 3 is the results of the identification of disease resistance in Example 2.
Fig. 4 is a schematic diagram of the structure of an RNAi interference vector.
Fig. 5 is the results of the identification of gene expression level in Example 3.
Fig. 6 is the results of the identification of disease resistance in Example 3.
Fig. 7 is the results of the correlation between the expression level of the *ZmPK* gene and the level of gray leaf spot.
Fig. 8 is a schematic diagram of the structure of a pCAMBIA3301 vector.
Fig. 9 is the results of the identification of gene expression level in Example 5.
Fig. 10 is the results of the identification of disease resistance in Example 5.
Fig. 11 is a schematic diagram of the structure of recombinant plasmid E and recombinant plasmid F.
Fig. 12 is the results of the identification of gene expression level in Example 6.
Fig. 13 is the results of the identification of disease resistance in Example 6.
Fig. 14 is the sequencing results of two gene-edited plants.
Fig. 15 is the results of the identification of disease resistance in Example 7.

### Description of the Preferred Embodiments

The following examples facilitate a better understanding of the present invention, but do not limit the present invention. The experimental methods in the following examples, unless otherwise specified, are all conventional methods. The experimental materials used in the following examples, unless otherwise specified, are all purchased from conventional biochemical reagent stores. The quantitative experiments in the following examples are all set to repeat the experiment three times, and the results are averaged.

The corn inbred line Y32 is a corn inbred line with high resistance to gray leaf spot of corn. The corn inbred line Y32 (line Y32) is described in the following documents: QTL mapping of resistance to gray leaf spot in maize, Yan Zhang ect., Theor Appl Genet DOI 10.1007/s00122-012-1954-z.

The corn inbred line Q11 is a corn inbred line highly susceptible to gray leaf spot. The corn inbred line Q11 (line Q11) is described in the following documents: QTL mapping of resistance to gray leaf spot in maize, Yan Zhang ect., Theor Appl Genet DOI 10.1007/s00122-012-1954-z.

The corn inbred line B73 (B73 inbred lines) is described in the following documents: The B73 maize genome: complexity, diversity, and dynamics, Patrick S. Schnable ect., Science (2009) 326:1112-1115. DOI: 10.1126/science. 1178534; The tin 1 gene retains the function of promoting tillering in maize, Zhang ect., Nature communictions (2019) 5608. DOI: 10.1038/s41467-019-134225-6.

The corn inbred line B73-329 (B73-329 inbred lines) is described in the following documents: A retrotransposon in an HKT1 family sodium transporter causes variation of leaf Na⁺ exclusion and salt tolerance in maize, Ming Zhang ect., New Phytologist (2018) 217: 1161-1176 doi: 10.1111/nph.14882.

*Cercospora zeina* is described in the following documents: First Report of Gray Leaf Spot of Maize Caused by *Cercospora zeina* in China, Plant Disease/Vol. 97 No. 12.

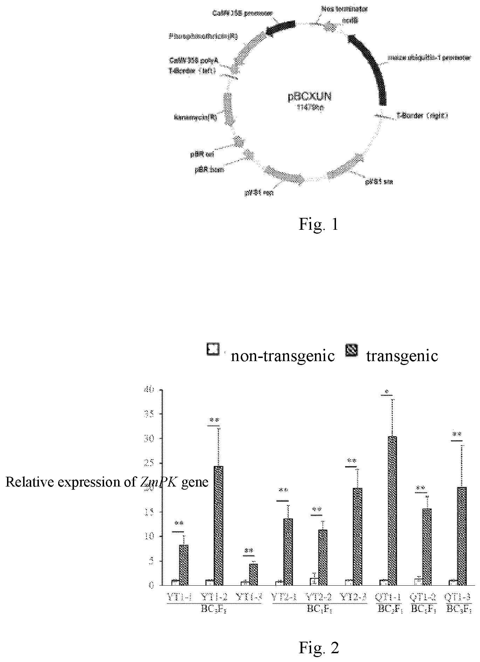

The pBCXUN vector is described in the following documents: ZmHAK5 and ZmHAK1 function in K⁺ uptake and distribution in maize under low K+ conditions. Journal of Intergrative Plant Biology (2018) doi:10.1111/jipb. 12756. The schematic diagram of the structure of a pBCXUN vector is shown in Fig.1.

The pGreen-HY104 vector (vector pGreen-HY104) is described in the following documents: A maize wall-associated kinase confers quantitative resistance to head smut, Nature Genetics (2015) 47:151-157.

The pCAMBIA3301 vector (bivalent expression vector *pCAMBIA3301*) is described in the following documents: Pyramiding of nine transgenes in maize generates high-level resistance against necrotrophic maize pathogens.

The pBUE411 vector is described in the following documents: ZmCCT9 enhances maize adaptation to higher latitudes. Huang ect., PNAS (2018) 115(2): E334-E341 DOI: 10.1073/pnas.1718058115.

### Example 1. Discovery of ZmPK protein and its encoding gene

The corn inbred line Y32 (as the donor parent) and the corn inbred line Q11 (as the recurrent parent) were used to construct the initial positioning population and the fine positioning population. The molecular markers located in the finely-located regions were used; the Y32BAC library of disease-resistant parents was screened by PCR. BAC clone fingerprint analysis was performed to construct BAC contigs covering the entire gene segment. The clone that could cover the least gene region was then selected for sequencing. A new gene was discovered through sequence alignment and expression analysis.

The *ZmPK* gene in the genomic DNA of the corn inbred line Y32 is shown in Sequence 1 in the sequence listing. There are two transcripts of the *ZmPK* gene. The first transcript encodes the protein shown in Sequence 2 in the sequence listing; the second transcript encodes the protein shown in Sequence 4 in the sequence listing. The open reading frame corresponding to the first transcript in the cDNA of the corn inbred line Y32 is shown in nucleotides 56-1618 in Sequence 3 in the sequence listing. The open reading frame corresponding to the second transcript in the cDNA of the corn inbred line Y32 is shown in nucleotides 56-1624 in Sequence 5 in the sequence listing.

The *ZmPK* gene in the genomic DNA of the three corn inbred lines (the three corn inbred lines refer to the corn inbred line B73, the corn inbred line B73-329 and the corn inbred line Q11, the same below) is as shown in Sequence 6 in the sequence listing. There are two transcripts of the *ZmPK* gene. The first transcript encodes the protein shown in Sequence 7 in the sequence listing. The second transcript encodes the protein shown in Sequence 9 in the sequence listing. The open reading frame corresponding to the first transcript of the cDNAs of the three corn inbred lines is shown in nucleotides 56-1618 in Sequence 8 in the sequence listing. The open reading frame corresponding to the second transcript of the cDNAs of the three corn inbred lines is shown in nucleotides 56-1624 in Sequence 10 in the sequence listing.

### Example 2. Obtaining and identifying over-expression plants

### I. Construction of recombinant expression vector

1. Fresh leaves of corn inbred line Y32 were taken, total RNA was extracted, and cDNA was obtained by reverse transcription.
2. The cDNA obtained in step 1 was used as a template; a primer pair composed of ZmPK-OE-F and ZmPK-OE-R was used for PCR amplification to obtain a PCR amplification product.
   ZmPK-OE-F: 5'-ATGGGCGCTTGCTTCTCCTC-3';
   ZmPK-OE-R: 5' -TCACAGAGCCTGAGGGTTTGG-3' .
3. The pBCXUN vector was taken, cleaved with restriction enzyme XcmI, and the vector backbone was recovered.
4. The PCR amplification product obtained in step 2 was linked to the vector backbone obtained in step 3 to obtain a recombinant plasmid A and a recombinant plasmid B. According to the sequencing results, the structure of recombinant plasmid A was described as follows: at the XcmI restriction site of the pBCXUN vector, a DNA molecule represented by nucleotides 56-1618 in Sequence 3 in the sequence listing was inserted. According to the sequencing results, the structure of recombinant plasmid B was described as follows: at the XcmI restriction site of the pBCXUN vector, a DNA molecule represented by nucleotides 56-1624 in Sequence 5 in the sequence listing was inserted. Since there were two sequences in the template and the difference was only 6 nucleotides, and the recombinant plasmid A and the recombinant plasmid B were constructed in the same way and produced at the same time, they needed to be identified by sequencing. In practical applications, two exogenous fragments can also be directly synthesized and then inserted into the XcmI restriction site of the pBCXUN vector to obtain a recombinant plasmid A and a recombinant plasmid B.
5. Fresh leaves of corn inbred line Q11 were taken, total RNA was extracted, and a cDNA was obtained by reverse transcription.
6. The cDNA obtained in step 5 was used as a template; a primer pair composed of ZmPK-OE-F and ZmPK-OE-R was used for PCR amplification to obtain a PCR amplification product.
7. The PCR amplification product obtained in step 6 was linked to the vector backbone obtained in step 3 to obtain a recombinant plasmid C. According to the sequencing results, the structure of recombinant plasmid C was described as follows: at the XcmI restriction site of the pBCXUN vector, a DNA molecule represented by nucleotides 56-1618 in Sequence 8 in the sequence listing was inserted.

### II. Acquisition of an overexpression plant

1. The recombinant plasmid was introduced into Agrobacterium EHA105 to obtain recombinant Agrobacterium.
2. The recombinant Agrobacterium obtained in step 1 was taken, and the Agrobacterium-mediated method was used to genetically transform the immature embryos of the corn inbred line B73-329 to obtain the T0 generation plants.
3. The T0 generation plants were identified by PCR.

The PCR identification method was as follows: plant leaves were taken, genomic DNA was extracted, and a primer pair consisting of bar-F and bar-R was used for PCR amplification. If an amplified product of about 262 bps was obtained, the PCR identification result was positive, and the plant was a transgenic plant. If no amplified product was obtained, the PCR identification result was negative, and the plant was a non-transgenic plant.
bar-F: GAAGGCACGCAACGCCTACGA; bar-R: CCAGAAACCCACGTCATGCCA.

The recombinant plasmid A was used for step two, and three randomly selected transgenic plants were named, namely YT1-1 plant, YT1-2 plant, and YT1-3 plant. The recombinant plasmid B was used to perform step two, and three randomly selected transgenic plants were named, namely YT2-1 plant, YT2-2 plant, and YT2-3 plant. The recombinant plasmid C was used for step two, and three randomly selected transgenic plants were named, namely QT1-1 plant, QT1-2 plant, and QT1-3 plant.

### III. Obtaining backcrossed separated offspring

The T0 generation plants were selfed, the seeds were harvested, and the seeds were then cultivated into plants, that is, the T1 generation plants. The transgenic plants were screened from the T1 generation plants. The PCR identification method was the same as 3 in step II. The T1 generation transgenic plant was used as the female parent, and the corn inbred line Q11 was used as the male parent. The seeds were cultivated into plants, that is, the F1 generation plants. The transgenic plants were screened from the F1 generation plants, and the PCR identification method was the same as 3 in step II. The F1 generation transgenic plant was used as the female parent, and the corn inbred line Q11 was used as the male parent. The seeds were cultivated into plants, that is, the BC₁F₁ generation plants. The transgenic plants and non-transgenic plants were screened from the BC₁ F₁ generation plants, and the PCR identification method was the same as 3 in step II. The BC₁ F₁ generation transgenic plant was used as the female parent, and the corn inbred line Q11 was used as the male parent. The seeds were cultivated into plants, that is, BC₂F₁ generation plants. The transgenic plants and non-transgenic plants were screened from the BC₂F₁ generation plants, and the PCR identification method was the same as 3 in step II. The BC₂F₁ generation transgenic plant was used as the female parent, and the corn inbred line Q11 was used as the male parent. The seeds were cultivated into plants, that is, BC₃F₁ generation plants. The transgenic plants and non-transgenic plants were screened from the BC₃F₁ generation plants, and the PCR identification method was the same as 3 in step II.

The T0 generation plants were: YT1-1 plant, YT1-2 plant, YT1-3 plant, YT2-1 plant, YT2-2 plant, YT2-3 plant, QT1-1 plant, QT1-2 plant, and QT1-3 plant.

### IV. Identification of plant disease resistance

The tested plants were: BC₃F₁ transgenic plants and non-transgenic plants of YT1-1 plants, BC₃F₁ transgenic plants and non-transgenic plants of YT1-2 plants, BC₃F₁ transgenic plants and non-transgenic plants of YT1-3 plants, BC₁F₁ generation transgenic plants and non-transgenic plants of YT2-1 plants, BC₁F₁ generation transgenic plants and non-transgenic plants of YT2-2 plants, BC₁F₁ generation transgenic plants and non-transgenic plants of YT2-3 plants, BC₂F₁ generation transgenic plants and non-transgenic plants of QT1-1 plants, BC₁F₁ generation transgenic plants and non-transgenic plants of QT1-2 plants, and BC₃F₁ generation transgenic plants and non-transgenic plants of QT1-3 plants.

### 1. Identification of gene expression level

The total RNA from the leaves of the tested plants was extracted and reverse transcribed to obtain the cDNA. With the GAPDH gene as the internal reference gene, the relative expression level of the *ZmPK* gene was detected by qPCR. The results are shown in Fig 2.

Primer pair used to detect *ZmPK* gene:
ZmPK-F: GCGTTGCCGTCAAGCGCAT; ZmPK-R:
GCTCCATCACAATGTACACGT.

Primer pair used to detect GAPDH gene:
GAPDH-F: ATCAACGGCTTCGGAAGGAT; GAPDH-R:
CCGTGGACGGTGTCGTACTT.

### 2. Identification of disease resistance

The disease resistance identification was carried out in Shangzhuang Experimental Base of China Agricultural University.

The pathogen of gray spot disease: *Cercospora zeina.*

The test plants were cultured under normal conditions, and pathogenic bacteria were inoculated during the bell mouth stage, and then the normal culture was continued. After two weeks of pollination, the phenotypic investigation was carried out, and the disease index (DSI) was calculated in a graded investigation. The specific method of inoculating pathogenic bacteria (bacterial fluid filling method) was as follows: the spores of the gray spot disease-causing fungus were added in sterile water to obtain a spore suspension with a spore concentration of 2-4×10⁵/mL, and a syringe was used to infuse the spore suspension into the corn bell mouth, and 5 ml were infused per corn plant.

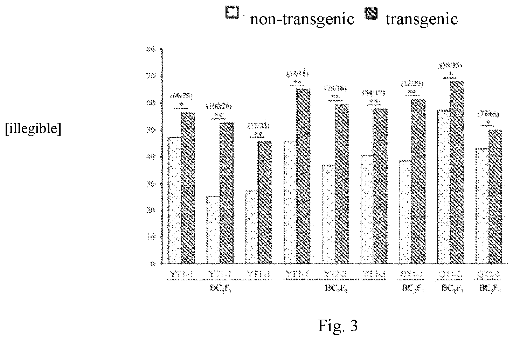

The grading standard of the disease level (X represents the percentage of the diseased spot area to the leaf area):
Level 1 (assigned value was 0): X≤5%;
Level 3 (assigned value was 0.25): 5%<X≤10%;
Level 5 (assigned value was 0.5): 10%<X≤30%;
Level 7 (assigned value was 0.75): 30%<X≤70%;
Level 9 (assigned value was 1): 70%<X≤100%.
Disease index (DSI) (%) = ∑ (assigned value corresponding to the disease level x the number of plants at this level) x 100/1 x total number of plants
The results are shown in Fig. 3. In Fig. 3, the parentheses indicate the number of plants, the number before the dividing line is the number of non-transgenic plants, and the number after the dividing line is the number of transgenic plants.

The results show that, compared with non-transgenic plants, the expression of *ZmPK* gene in transgenic plants was significantly higher, and the disease index of corresponding transgenic plants was also significantly higher.

### Example 3. Obtaining and identifying plants in which the expression is inhibited

### I. Obtaining plants in which the expression is inhibited

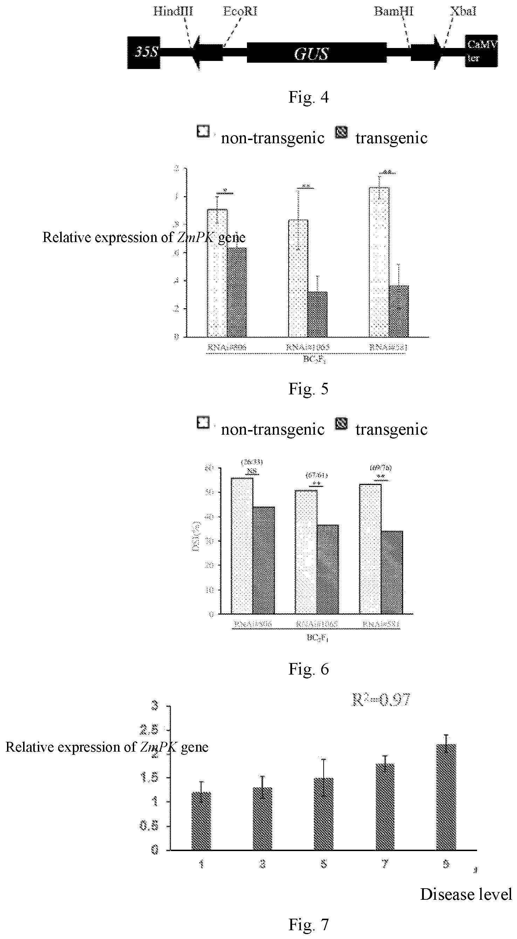

1. The pGreen-HY104 vector was used as the starting vector, the forward fragment was inserted between the *BamH*I and *Xba*I restriction sites, and the reverse fragment was inserted between the *Hind*III and *EcoR*I restriction sites, so as to obtain an RNAi interference vector. The RNAi interference vector was verified by sequencing. The forward segment and the reverse segment were in a reverse complementary relationship. The forward fragment is shown in Sequence 11 in the sequence listing. The structure diagram of the RNAi interference vector is shown in Fig. 4.
2. The RNAi interference vector obtained in step 1 was introduced into Agrobacterium EHA105 to obtain recombinant Agrobacterium.
3. The recombinant Agrobacterium obtained in step 2 was taken, and the Agrobacterium-mediated method was used to genetically transform the immature embryos of the corn inbred line B73-329 to obtain the T0 generation plants.
4. The T0 generation plants were selfed, the seeds were harvested, and the seeds were then cultivated into plants, that is, the T1 generation plants.
5. The T0 generation plants were identified by PCR.

The PCR identification method was the same as 3 in step II in Example 2.

Three randomly selected transgenic plants were named as RNAi#806 plants, RNAi#1065 plants, and RNAi#581 plants.

### II. Obtaining backcrossed separate population

The PCR identification method was the same as 3 in step II in Example 2.

The T0 generation plants were selfed, the seeds were harvested, and the seeds were then cultivated into plants, that is, the T1 generation plants. The transgenic plants were screened from the T1 generation plants (PCR identification). The T1 generation transgenic plant was used as the female parent, and the corn inbred line Q11 was used as the male parent. The seeds were cultivated into plants, that is, the F1 generation plants. The transgenic plants were screened from the F1 generation plants (PCR identification). The F1 generation transgenic plant was used as the female parent, and the corn inbred line Q11 was used as the male parent. The seeds were cultivated into plants, that is, the BC₁F₁ generation plants. The transgenic plants and non-transgenic plants were screened from the BC₁F₁ generation plants (PCR identification). The BC₁F₁ generation transgenic plant was used as the female parent, and the corn inbred line Q11 was used as the male parent. The seeds were cultivated into plants, that is, BC₂F₁ generation plants. The transgenic plants and non-transgenic plants were screened from the BC₂F₁ generation plants (PCR identification).

The T0 generation plants were: RNAi#806 plant, RNAi#1065 plant, and RNAi#581 plant.

### III. Identification of plant disease resistance

The tested plants were: BC₂F₁ transgenic plants and non-transgenic plants of RNAi#806 plants, BC₂F₁ transgenic plants and non-transgenic plants of RNAi#1065 plants, and BC₂F₁ transgenic plants and non-transgenic plants of RNAi#581 plants.

### 1. Identification of gene expression level

The method was the same as 1 in step IV in Example 2.

The results are shown in Fig. 5.

### 2. Identification of disease resistance

The method was the same as 2 in step IV in Example 2.

The results are shown in Fig. 6. In Fig. 6, the parentheses indicate the number of plants, the number before the dividing line is the number of non-transgenic plants, and the number after the dividing line is the number of transgenic plants.

The results show that, compared with non-transgenic plants, the expression of *ZmPK* gene in transgenic plants was significantly reduced, and the disease index of the corresponding transgenic plants was significantly reduced.

### Example 4. Analysis of the correlation between gene expression and disease resistance

The original parents of the positioning population were corn inbred line Y32 and corn inbred line Q11. Through continuous crossing and backcrossing, a high-generation backcrossing segregation population was constructed.

Three plants with disease levels of 1, 3, 5, 7, and 9 in the positioning population were randomly selected, and gene expression level identification (the method was the same as that of 1 in step IV in Example 2) and disease resistance identification (the method was the same as 2 in step IV in Example 2) were performed. The correlation between the expression of *ZmPK* gene and the level of gray leaf spot was calculated. The results are shown in Fig. 7. There was a significant positive correlation between the expression of *ZmPK* gene and the level of gray leaf spot. This further confirms the negative correlation between *ZmPK* gene expression and plant resistance to gray leaf spot.

### Example 5. Obtaining and identifying complementary transgenic plants

### I. Construction of recombinant expression vector

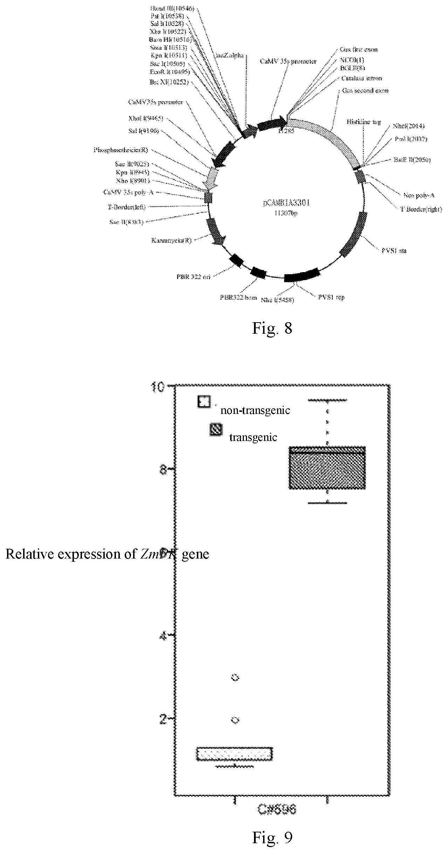

A recombinant plasmid was prepared. According to the sequencing results, the structure of the recombinant plasmid was described as follows: the DNA molecule shown in Sequence 12 in the sequence list was inserted into the *BamH*I restriction site of the pCAMBIA3301 vector. Fig. 8 shows a schematic diagram of the structure of the pCAMBIA3301 vector.

### II. Obtaining complementary transgenic plants

1. The recombinant plasmid prepared in step I was introduced into Agrobacterium EHA105 to obtain recombinant Agrobacterium.
2. The recombinant Agrobacterium obtained in step 1 was taken, and the Agrobacterium-mediated method was used to genetically transform the immature embryos of the corn inbred line B73 to obtain the T0 generation plants.
3. The T0 generation plants were identified by PCR.

The PCR identification method was the same as 3 in step II in Example 2.

A randomly selected transgenic plant was named C#596 plant.

### III. Obtaining backcrossed separated offspring

The T0 generation plants were selfed, the seeds were harvested, and the seeds were then cultivated into plants, that is, the T1 generation plants. The transgenic plants were screened from the T1 generation plants. The PCR identification method was the same as 3 in step II. The T1 generation transgenic plant was used as the female parent, and the corn inbred line Q11 was used as the male parent. The seeds were cultivated into plants, that is, the F1 generation plants. The transgenic plants were screened from the F1 generation plants, and the PCR identification method was the same as 3 in step II. The F1 generation transgenic plant was used as the female parent, and the corn inbred line Q11 was used as the male parent. The seeds were cultivated into plants, that is, the BC₁F₁ generation plants.

### IV. Identification of plant disease resistance

The tested plants were: BC₁F₁ generation transgenic plants and non-transgenic plants of C#595 plants, and BC₁F₁ generation transgenic plants and non-transgenic plants of C#596 plants.

### 1. Identification of gene expression level

The method was the same as 1 in step IV in Example 2.

The results are shown in Fig. 9.

### 2. Identification of disease resistance

The method was the same as 2 in step IV in Example 2.

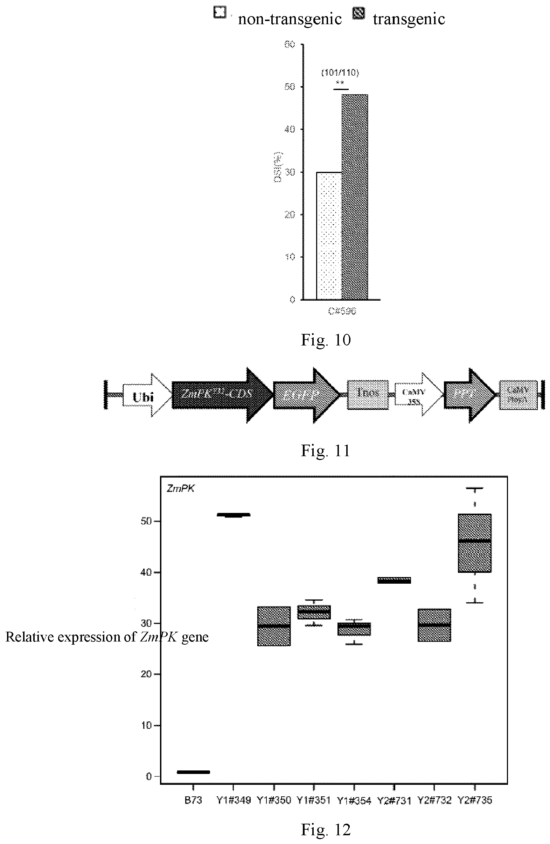

The results are shown in Fig. 10. In Fig. 10, the parentheses indicate the number of plants, the number before the dividing line is the number of non-transgenic plants, and the number after the dividing line is the number of transgenic plants.

The results show that, compared with non-transgenic plants, the expression of *ZmPK* gene in transgenic plants was significantly higher, and the disease index of corresponding transgenic plants was also significantly higher.

### Example 6 Obtaining and identifying pure lines of fusion overexpression plants 1. Construction of recombinant expression vector

The recombinant plasmid E and recombinant plasmid F were prepared respectively. According to the sequencing results, the structure of recombinant plasmid E was described as follows: at the *Xcm*I restriction site of the pBCXUN vector, a DNA molecule shown in Sequence 13 of the sequence listing was inserted. According to the sequencing results, the structure of recombinant plasmid F was described as follows: at the *Xcm*I restriction site of the pBCXUN vector, a DNA molecule shown in Sequence 14 of the sequence listing was inserted. The schematic diagram of the structure of recombinant plasmid E and recombinant plasmid F is shown in Fig. 11.

### II. Obtaining pure lines of fusion overexpression plants

1. The recombinant plasmid was introduced into Agrobacterium EHA105 to obtain recombinant Agrobacterium.
2. The recombinant Agrobacterium obtained in step 1 was taken, and the Agrobacterium-mediated method was used to genetically transform the immature embryos of the corn inbred line B73 to obtain T0 generation plants.
3. The T0 generation plants were identified by PCR.

The PCR identification method was the same as 3 in step II of Example 2.

The recombinant plasmid E was used to carry out step II, and four randomly selected transgenic plants were named: Y1#349 plant, Y1#350 plant, Y1#351 plant, and Y1#354 plant. The recombinant plasmid F was used to carry out step II, and three randomly selected transgenic plants were named, namely Y2#731 plant, Y2#732 plant, and Y2#735 plant.

4. The T0 generation plants were selfed and the seeds were harvested. The seeds were cultivated into plants, which were the T1 generation plants. The transgenic plants (identified by PCR) were screened from the T1 generation plants for selfing, and the seeds were harvested. The T2 generation plants were cultivated from the seeds into the plants. The transgenic plants (identified by PCR) from the T2 generation plants were screened for selfing, and the seeds were harvested. The seeds were cultivated to plants, that is, the T3 generation plants. The PCR identification method was the same as 3 in step II in Example 2. For a T2 generation plant, if the T3 generation plants obtained by selfing were all transgenic plants, the T2 generation plant was a homozygous transgenic plant. The offspring obtained by selfing of the homozygous transgenic plant was a homozygous transgenic line.

The following homozygous transgenic strains were obtained: Y1#349 strain, Y1#350 strain, Y1#351 strain, Y1#354 strain, Y2#731 strain, Y2#732 strain, and Y2#735 strain.

### III. Identification of plant disease resistance

The tested plants were: T3 generation plants of Y1#349 line, T3 generation plants of Y1#350 line, T3 generation plants of Y1#351 line, T3 generation plants of Y1#354 line, T3 generation plants of Y2#731 line, T3 generation plants of Y2#732 line, T3 generation plants of Y2#735 line, and the corn inbred line B73 plants.

### 1. Identification of gene expression level

The method was the same as 1 in step IV in Example 2.

The results are shown in Fig. 12.

### 2. Identification of disease resistance

The method was the same as 2 in Step IV in Example 2.

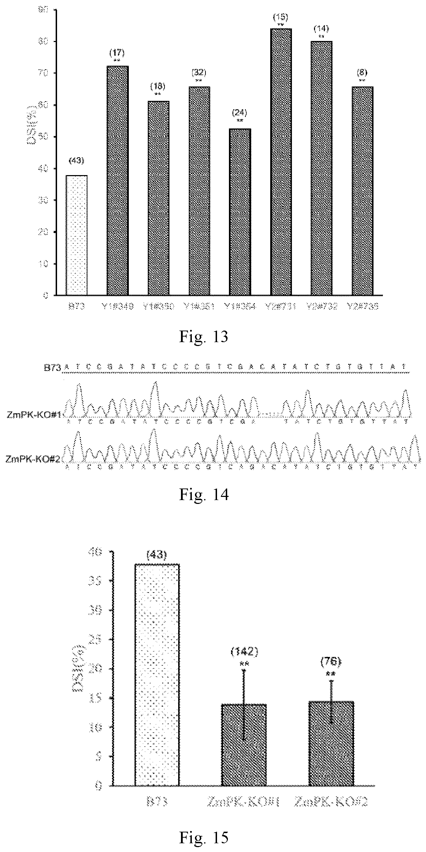

The results are shown in Fig. 13. In Fig. 13, the parentheses indicate the number of plants.

The results show that compared with the corn inbred line B73 plants (control non-transgenic plants), the expression of *ZmPK* gene in the transgenic plants was significantly higher, and the disease index of the corresponding transgenic plants was also significantly higher.

### Example 7 Obtaining and identifying gene-edited plants

### 1. Construction of gene editing vector

A gene editing vector was prepared, that is, a recombinant plasmid was obtained by: inserting the double-stranded DNA molecule shown in Sequence 16 in the sequence listing into the *Bsa*I restriction site of the pBUE411 vector. The recombinant plasmid was verified by sequencing. The gene editing vector encoded two sgRNAs, the target sequence binding region of one sgRNA is shown in Sequence 17 in the sequence listing, and the target sequence binding region of the other sgRNA is shown in Sequence 18 in the sequence listing. The gene editing vector contained the Cas9 gene and expressed the Cas9 protein.

### II. Obtaining gene-edited plants

1. The gene editing vector was introduced into Agrobacterium EHA105 to obtain recombinant Agrobacterium.
2. The recombinant Agrobacterium obtained in step 1 was taken, and the Agrobacterium-mediated method was used to genetically transform the immature embryos of the corn inbred line B73 to obtain T0 generation plants.
3. The plants with mutations in the target sequence were screened from the T0 generation plants.

The specific method was as follows: the plant leaves were taken, the genomic DNA was extracted, a primer pair composed of F and R was used for PCR amplification, and the PCR amplification product was recovered and sequenced. The sequencing result was compared with the wild-type sequence (the wild-type sequence is shown in the 9599-10985 in Sequence 12 in the sequence listing), and plants with different sequences were screened.
F: 5'-TTGAGGTCATTGTCTCAGCC-3';
R: 5' -AGCAGCTTGGCAGCGTATG-3'.

4. The T0 generation plants screened in step 3 were taken, and used for selfing and then harvested to obtain the seeds. The seeds were cultivated into plants, which were the T1 generation plants.

5. The gene-edited plants were screened from T1 generation plants.
(1) The plants were subjected to PCR identification with the bar gene in the gene editing vector as the target. PCR identification method was as follows: plant leaves were taken, genomic DNA was extracted, and the primer pair containing bar-F and bar-R was used for PCR amplification. If no amplified product was obtained, the PCR identification result was negative.
(2) The plants with the target region of gene editing were identified as the target. The identification method was the same as step 3.

If a plant is identified as negative by PCR in step (1), and the identification result in step (2) shows that it is different from the wild-type sequence and is homozygous (and the two chromosomes are identical), the plant is a gene-edited plant.

Two gene-edited plants were obtained, named ZmPK-KO#1 plant and ZmPK-KO#2 plant, respectively.

The sequencing results of step (2) of the two gene editing plants are shown in Fig. 14. Compared with B73, two nucleotides were missing in the ZmPK-KO#1 plant, and a nucleotide was inserted into the ZmPK-KO#2 plant. Both caused frameshift mutations.

6. The gene-edited plants were taken, self-bred, and offspring plants were obtained, that is, the gene-edited plants.

Two gene editing lines were obtained, and named ZmPK-KO#1 strain and ZmPK-KO#2 strain, respectively.

### 3. Identification of disease resistance of gene-edited plants

The tested plants were: T2 generation plants of ZmPK-KO#1 line, T2 generation plants of ZmPK-KO#2 line, and corn inbred line B73 plants.

The method was the same as 2 in step IV in Example 2.

The results are shown in Fig. 15. In Fig. 15, the parentheses indicate the number of plants.

Compared with corn inbred line B73 plants, the disease index of gene-edited plants was significantly reduced.

### Industrial applicability

The present invention provides a major gene *ZmPK* for resistance to gray leaf spot of corn. Through transgene complementation, overexpression, CRISPR knockout and RNAi interference experiments, the gene's anti-grey leaf spot function-negative regulation of gray leaf spot resistance has been proved. The present invention has great application value for the breeding of corn against gray leaf spot.

## Claims

1. A protein, which is as follows: (a1), or (a2), or (a3), or (a4), or (a5), or (a6), or (a7), or (a8):
(a1) a protein represented by SEQ ID NO: 2 in the sequence listing;
(a2) a protein represented by SEQ ID NO: 4 in the sequence listing;
(a3) a protein represented by SEQ ID NO: 7 in the sequence listing;
(a4) a protein represented by SEQ ID NO: 9 in the sequence listing;
(a5) a fusion protein obtained by attaching a tag to an N-terminus or/and a C-terminus of the protein in any one of (a1) to (a4);
(a6) a protein comprising the following three segments from N-terminus to C-terminus: the protein in any one of (a1) to (a4), a connecting peptide, and an EGFP protein;
(a7) a protein related to plant gray leaf spot resistance obtained by substituting and/or deleting and/or adding one or a plurality of amino acid residues to the protein in any one of (a1) to (a6); and
(a8) a protein related to plant gray leaf spot resistance obtained from corn and having a homology of 90%, 91%, 92%, 93%, 94%, 95%, 96%, 97%, 98% or 99% with the protein in any one of (a1) to (a4).

2. A nucleic acid molecule, which encodes the protein according to claim 1.

3. The nucleic acid molecule according to claim 2, **characterized in that**: the nucleic acid molecule is any one of the following (b1) to (b15):
(b1) a DNA molecule with an encoding region that is represented by nucleotides 56 to 1618 in SEQ ID NO: 3 in the sequence listing;
(b2) a DNA molecule represented by SEQ ID NO: 3 in the sequence listing;
(b3) a DNA molecule with an encoding region that is represented by nucleotides 56 to 1624 in SEQ ID NO: 5 in the sequence listing;
(b4) a DNA molecule represented by SEQ ID NO: 5 in the sequence listing;
(b5) a DNA molecule represented by SEQ ID NO: 1 in the sequence listing;
(b6) a DNA molecule with an encoding region that is represented by nucleotides 56 to 1618 in SEQ ID NO: 8 in the sequence listing;
(b7) a DNA molecule represented by SEQ ID NO: 8 in the sequence listing;
(b8) a DNA molecule with an encoding region that is represented by nucleotides 56 to 1624 in SEQ ID NO: 10 in the sequence listing;
(b9) a DNA molecule represented by SEQ ID NO: 10 in the sequence listing;
(b10) a DNA molecule represented by SEQ ID NO: 6 in the sequence listing;
(b11) a DNA molecule represented by SEQ ID NO: 12 in the sequence listing;
(b12) a DNA molecule represented by SEQ ID NO: 13 in the sequence listing;
(b13) a DNA molecule represented by SEQ ID NO: 14 in the sequence listing;
(b14) a DNA molecule that is derived from corn, has a homology of 90%, 91%, 92%, 93%, 94%, 95%, 96%, 97%, 98% or 99% with any one of (b1) to (b13), and encodes the protein;
(b15) a DNA molecule that hybridizes to any one of (b1) to (b13) under a stringent condition, and encodes the protein.

4. An expression cassette, a recombinant vector or a recombinant microorganism comprising the nucleic acid molecule according to claim 2 or 3.

5. An application of the protein according to claim 1, which is the following (c1) or (c2):
(c1) to regulate the resistance of a plant to gray leaf spot;
(c2) to reduce the disease resistance of a plant to gray leaf spot.

6. The application according to claim 5, **characterized in that** the plant is a plant of the genus Zea.

7. An application of the nucleic acid molecule according to claim 2 or 3, which is the following (d1) or (d2):
(d1) to cultivate a transgenic plant with altered resistance to gray leaf spot;
(d2) to cultivate a transgenic plant with reduced resistance to gray leaf spot.

8. The application according to claim 7, **characterized in that** the plant is a plant of the genus Zea.

9. An application of a substance for inhibiting an activity of the protein according to claim 1 in a plant and/or for reducing an abundance of the protein according to claim 1 in a plant to enhance the disease resistance of the plant to gray leaf spot.

10. The application according to claim 9, **characterized in that** the plant is a plant of the genus Zea.

11. An application of a substance for inhibiting a transcription of the nucleic acid molecule according to claim 2 or 3 and/or for inhibiting an expression of the nucleic acid molecule according to claim 2 or 3 and/or for gene editing of the nucleic acid molecule according to claim 2 or 3 to enhance the disease resistance of the plant to gray leaf spot.

12. The application according to claim 11, **characterized in that** the plant is a plant of the genus Zea.

13. A method for preparing a transgenic plant, comprising the steps of: introducing the nucleic acid molecule according to claim 2 or 3 into a starting plant to obtain a transgenic plant with reduced gray leaf spot resistance.

14. The application according to claim 13, **characterized in that** the starting plant is a plant of the genus Zea.

15. A plant breeding method, comprising the following steps: increasing a content and/or activity of the protein according to claim 1 in a target plant, thereby reducing the gray leaf spot resistance of the target plant.

16. The application according to claim 15, **characterized in that** the target plant is a plant of the genus Zea.

17. A method for preparing a transgenic plant, comprising the following steps: inhibiting an expression of the nucleic acid molecule according to claim 2 or 3 in a starting plant to obtain a transgenic plant with increased gray leaf spot resistance.

18. The application according to claim 17, **characterized in that** the starting plant is a plant of the genus Zea.

19. A plant breeding method, comprising the following steps: reducing a content and/or activity of the protein according to claim 1 in a target plant, thereby increasing the disease resistance of the target plant to gray leaf spot.

20. The application according to claim 19, **characterized in that** the target plant is a plant of the genus Zea.

21. A plant breeding method, comprising the steps of: performing gene editing on a specific gene in a genome of a starting plant to increase the gray leaf spot resistance of the target plant; and the specific gene encoding the protein according to claim 1.

22. The application according to claim 21, **characterized in that** the starting plant is a plant of the genus Zea.


SEQUENCE LISTING

<110>  China Agricultural University

<120>  METHOD FOR CULTIVATING PLANT RESISTANT TO GRAY LEAF SPOT

<130>  GNPYX20003

<160>  19

<170>  PatentIn version 3.5

<210>  1
<211>  6946
<212>  DNA
<213>  Zea mays L.

<400>  1
gcaaatccat atgctcagct cccgcctcct cccatccccg gaccccggac cccggccatg      60

ggcgcttgct tctcctccgc ctctgccgcc cccgccggcg ccgccgtcga cgagcgccgc     120

ccgtccaagg agggcgacgg caagaagagg cgccgcgccg ccggggcatc gccggatgcc     180

gcggcgcccg tgcgcgtgga gttcggctac gagagggact tcgaggcgcg ctacgaggtc     240

ggccgcctgc tcggccacgg ccagttcggc tacaccttcg ccgccaccga ccgcggctct     300

ggggaccgcg ttgccgtcaa gcgcatcgac aaggccaagg tgagctgccg cctgcccccc     360

cgcaccccaa gccgccgcgc tgtccctgtc tctgtctctc ctactagtag tagtagctgg     420

tggtgattcc gagcgcgtct ttggtctggt gcatcgaacc acttgtgctt ggtgcatttc     480

gaggggattc ggtgtaattc cgtgcaaatt ggggatttct ctcctgttgc tttccgaggt     540

ttaggtgttt cgattgggac gcgattggag ccgttcattt taggacattt ccggtgcctt     600

ttgggaggcg tttagctcaa cgagtagctc actcacattt ctagctgttt ggccgcttca     660

tttctcccaa gctttcgttg tttgccggtg gttctgagct gcgggatctt gacgttggcc     720

agagaggtgg tttcgacatt caggcatctc ggatgacctc ttagtttggc actacagctc     780

tattatttcg ggaacgacgt gttgctcagt gcgcacctca ttcatggaag tggcaaggtc     840

gcttgtctgc agaacgggga aggtgctttt catctggcta ttcatggaaa acgacttgtt     900

cagttgccct actaataatt tcaataagat tgcctgcctc cttgaatggt tggggcttgg     960

aaggttcctg tcgaagaaaa agtcaggaaa gataacaatt gcgcacttgc agtggacaac    1020

gcttccctgt cttctatgct ataggtggac agcatttttc taggtataat taatttgacc    1080

ttcaaacata tgtatactaa ccaacgcggt tttgattcca tcaaatgttt tggactctct    1140

ctgctgaact gtcaaagtta cttcatgggg caaaatgtca aattttctgg aaccttccgt    1200

agtatatttt ggaaatgagt gtttattgtg tcattggaaa taccgttcat gtgtctgtga    1260

cagaatgtgt cactagaaag ctgaattggt gttgtccttg tcaaaaaggc actaaacacg    1320

agtctgaaaa ttaggcctgt tcttggtaag ggaaggaatc tgagcatcaa tgctgatagg    1380

aatagactct gtctgtcaat attgttaact tgtttatagg gcttcgagtt ttcaactttt 1440

gaggcagata agtaggatac ctcttttgat catgatatat aacatattct tatatacctc 1500

aagccttgca ctgttaagtt aatgtggcat cctttctaga gatcatgacc tcaagttgca 1560

tatggatgcc aataatatcg acaccaagtg aacatcagtg tctgtggaat atgccgaaag 1620

cagccaacgt gccattactg aattttcata tgattattat attctgttta gatttattta 1680

cgtcggaaca cagtgagatg gtaacgtaat gaatcaaaat aggctataaa catgcaattc 1740

aacatatcat tatcatgccc aagtgttttg tcattctatc tttattcgtc caagaaggac 1800

aagcctggtg cattgttgag ggaaccagtt cttctgcagt acttctaggg aggtaaaaat 1860

tcaacaccgt tggatgcaga tctatcgaac ccagggactt tgtgcttcca gtgaaaagtt 1920

atatggaccc ataggccaga ggatgtgaga gttttacctc tctggaagtt atatgcgcta 1980

gcattagtgt ggtcatcaat gggatcaaag atgagctcca cctttggtgt agagctggag 2040

ctaggggact ctagcatcct ggcgctccaa tcttccatcc agtgaactct gttttttggg 2100

tctagtaggt caagggtcca gttatttttt ctttctgctg taaagtctct agttaaggtg 2160

tgagttttgt atggtgtttt ttcgaggttt ccccaaacct cacctttttt ccttcttaat 2220

ataatgatat gcagctttcc tgcgtattcg agaaaagaaa gttttatctc tctggaagtt 2280

aactgcagag gaacttgtta cattgttgag agttgtctca ccgagtcacc aggtcgctgg 2340

ttcaaagcag tctctccaca tttatgtgga aggcttgcct cggtttatcc cttcccaaga 2400

ctctacttgt gggagactct ggcattgggt ctgtcctatg ccgttgaagc gctaggttcg 2460

tttatccctt ccctataccc acttgtcaga gcctccaaca ctgagtctgc cctaagcttc 2520

caagttccaa cactgggtct gccctaggcc gtcgaagcgt tatatgattg ccatgtactg 2580

ttatgctttg ttgccttcac atattttccg ttcgaaatca tctccttgtt gccttcacat 2640

attgccttgt tgctttcaca tattttctgt tccacgtcat acttagaagt tagaacacgt 2700

gatttatgcc aattaagatt attattttat ataacagatg acccgccctg ttgctgtgga 2760

ggatgtgaaa agagaagtga agattcttaa agcacttaaa ggacatcaga atattgttca 2820

cttctacaat gcatttgagg atgattcata cgtgtacatt gtgatggagt aagtaggccc 2880

atacacctgt tcctgctaat agagcatatc gattttgcta tgactttttt ccctaaagtt 2940

ttaacatgaa caatatctat cctgtttaca gaatcctaga cactaaaatg tcatttctaa 3000

ttatcaatta ttctatagct aaaccagatg caatcctgat ttatttttct taacgtatgg 3060

atatattgga cttttctttc aaacctgcat tttgaatttg attacaggga actataacac 3120

taattcagaa ctctatcatg tttaacattt ttcttgcatt gttctatgtt tgtcaacttg 3180

acgcacttct tagataatat aacatcatct tccacagtca ccattagtta ggaccttgga 3240

ccttcatggt tccgaaattt agctaagaat ggtatacatg gtcatgtgat ttcaaataga 3300
tgttcctata tgccagaacc aactcataag tcataagttt taccttgtgt ttttgcaggc 3360
tatgtgaggg cggtgaacta ttagatcgga ttttggcaaa gtaagtagat aagatcccca 3420
tctctttgtt tcccgtacct cattcttcgc cattaaattt atagattttt gtgctgtaaa 3480
atcagattgc tttatgttgt ttgtctgctt tgtttgattt ctagttgctc gttcaagatc 3540
ctttacttaa tggtgtgcgt gttttgacag aaagaatagc cgctatagtg agaaagatgc 3600
tgcagtggta gtccgccaaa tgctcaaagt agctgctgaa tgccatctgc gtgggttagt 3660
tcaccgagat atgaagcctg aggtagaaat caaatacttc aatctctttg cacacagtaa 3720
gcatttggtg atatttcact acttcctcag gtcatgtaag actgtaccta ttttccttcc 3780
cagaacttcc ttttcaaatc gaacaaggag gattcaccac taaaggcgac agattttggt 3840
ttgtcagatt tcattaagcc aggtatctac ttggggccat ctgaatctgt cgggaatctg 3900
ataggggcaa gtctgcagtt tagctgacca ttttgttgtc taatgcatgc tttagggaag 3960
aagttccatg acattgttgg aagtgcttac tatgtcgcac cagaagtact aaaacgacgg 4020
tctggtcctg agtcagatgt ttggagcata ggagtcataa cctacatttt gctctgtggg 4080
aggcgccctt tttgggataa gaccgaagac ggtatattca aggaggtaag tggatggatt 4140
ttgcatacca tgtgcttaca tgtaaaatat gcttggttag agtgctgtac cagggatcag 4200
cgttttcagc gtgctgatac tgttttgtac aatgtgtttc tactttctac gtcatatagc 4260
agtgtttctt tgttaactat ttcagtgtca aactatttgt cgtgtcacaa ctcagcagta 4320
taattttact attttgaaca ctgtaaacct gcctggtcag gttatccttc agtaatttct 4380
ctactagcta ccagaaaccc actttatgca ggtgttcagt ttaataacac ccaccatctt 4440
tcagatttct aatgttcagt gttagacaga cttcattaag atgcacctta agatgattgt 4500
aagtagtaaa agtgctttgc acttttgtta acttttgagt ctgaagatga cttgtggtac 4560
ctatgacctc aagaaaccaa ggcattgcca ttggaatagc taattcgaat gagcttcaga 4620
tatggctatc tgttttagtt ttggacatct gactcaactt tataggataa tactatatta 4680
gcaatctttg aggtcattgt ctcagccaaa ataagttgcg gtctcttttt tactgtccta 4740
agcagcaata tggtttccat tttcattata ccagcaactt ccaccttttt cttgctattt 4800
aaatatcttt atgcatttta tcagcaagga catgatacga tcgtatatgt gatattctac 4860
atcttttcac ttctcataat taggttctaa ggaacaagcc tgattttcgt aagaggcctt 4920
ggtcaagcat cagcccaggt gctaaagatt ttgttaaaag gttactagtg aagaatccaa 4980
gggccaggct aacagctgct caagctctct gtaagttttg gtatttttca ttaatttact 5040
agcctagtca tgatgatcag attcaccttc tctatgtgag aacagagaac acatatacat 5100
ctggcagtat gcctttcaat cagttatgac aatgtaaata tgcaaagacc gatgtttttt 5160

ctatcctgca ccattttaga acattaatgg ggaaaaacca caatatatta ggaaaaatgt 5220

ttaattatgt cctggtcact tgaaatgaac atataccact gaggttttct agttctcatg 5280

cgttcttata atgatctaat aagtcagtgg aggtttgctg cccaccaccc ctacatttgt 5340

attgtgaatt actatcatct ttactgatcc tgattgttct tgatatgtta agcacatccg 5400

tgggtaagag aaggagggga agcatccgat atccccgtcg acatatctgt gttatcaaac 5460

atgcgtcagt ttgtcaagta cagccgtttc aagcaattcg cgcttcgggt aattacagtg 5520

attacaaaaa acaacactgc atcgtttatt ttttcctcac aatatttcct cgtggcatgg 5580

tcaggctctg gcgagcaccc ttaacgagga agagctatca gatctgaagg atcagtttga 5640

tgcaattgat atcgataaaa gtggatcgat tagtatcgag gaaatgcgtc atgtaggttc 5700

tgttagtgtt tgctgatgaa aatgccttag atcctgaact actctgcggt gctgattaat 5760

ctgtgcatgt ttcggtaggc ccttgcaaag gatcttccct ggagattgaa gggtccccgt 5820

gtgctggaga ttattcaagc agtaagtttg agccttcttc tggatccagc cctttctttg 5880

ttacccccct tgtttccaag aaaatagctg gccttgttct gagggtataa ccaaaactgc 5940

atcttatttt gtggtagatt gacagcaaca ctgatgggct cgtggacttc aaggagtttg 6000

ttgcggcaac tctccatatc caccagatgg cggagctcga ctcagaaagg tggggcatac 6060

gctgccaagc tgctttcagt aagtttgatc ttgacggtga tggatatatc acgccggagg 6120

aactcagaat ggtaattttc tactcctgtc ttgtttccat gttgcttcac caacgaatgc 6180

acagttcaca taacccttat tatcatcact gcttcccatg aataactagc tggctcgacc 6240

atcatgagat tcagtacttg cgccctgtgc acttggtttt ggtcccgctt gttagaatga 6300

agtaatttat caatggaagc gctgtaatat tttaatcagc gtttagattt gataaagata 6360

aaacatgttc attgtttgtg ccaagaaatc cacttacaca gatactgaga gttgcaccgt 6420

agataacgct aatcggcagt atcctaatcg agattttctt tcaaggtgca gcaccctggg 6480

ttgaagggat ctatcgagcc gctgctggag gaggccgaca tcgacaaaga cggcaagata 6540

agcctgtccg agttccgcaa gctcctacgg acagcgagca tgagcaacgt acccagccca 6600

agggggcccc caaaccctca ggctctgtga attccggctc ggccactagg gaggagcaag 6660

cttaggaagt tgccatacaa tagccatgtg ttctttgggt tcttcagagt gccatgtgat 6720

gtttctggtt tttagcatcc aggttatgtg tgcagtgcag ccccgagtga gtttcgaagt 6780

aaatattcag tgctttcttt ttctttccgg aagagtgaga ggtggaggtc aaaatggtag 6840

gcaagactcg ccttcttctt tcctttacac tgtacagtga tactgaaata tgtacgattt 6900

ttattataac tgttcgtcgc aataaagtta tttggagaag tgagga 6946

<210> 2

<211>  520
<212>  PRT
<213>  Zea mays L.

<400>  2

Met Gly Ala Cys Phe Ser Ser Ala Ser Ala Ala Pro Ala Gly Ala Ala
1               5                   10                  15

Val Asp Glu Arg Arg Pro Ser Lys Glu Gly Asp Gly Lys Lys Arg Arg
            20                  25                  30

Arg Ala Ala Gly Ala Ser Pro Asp Ala Ala Ala Pro Val Arg Val Glu
        35                  40                  45

Phe Gly Tyr Glu Arg Asp Phe Glu Ala Arg Tyr Glu Val Gly Arg Leu
    50                  55                  60

Leu Gly His Gly Gln Phe Gly Tyr Thr Phe Ala Ala Thr Asp Arg Gly
65                  70                  75                  80

Ser Gly Asp Arg Val Ala Val Lys Arg Ile Asp Lys Ala Lys Met Thr
                85                  90                  95

Arg Pro Val Ala Val Glu Asp Val Lys Arg Glu Val Lys Ile Leu Lys
            100                 105                 110

Ala Leu Lys Gly His Gln Asn Ile Val His Phe Tyr Asn Ala Phe Glu
        115                 120                 125

Asp Asp Ser Tyr Val Tyr Ile Val Met Glu Leu Cys Glu Gly Gly Glu
    130                 135                 140

Leu Leu Asp Arg Ile Leu Ala Lys Lys Asn Ser Arg Tyr Ser Glu Lys
145                 150                 155                 160

Asp Ala Ala Val Val Val Arg Gln Met Leu Lys Val Ala Ala Glu Cys
                165                 170                 175

His Leu Arg Gly Leu Val His Arg Asp Met Lys Pro Glu Asn Phe Leu
            180                 185                 190

Phe Lys Ser Asn Lys Glu Asp Ser Pro Leu Lys Ala Thr Asp Phe Gly
        195                 200                 205

Leu Ser Asp Phe Ile Lys Pro Gly Lys Lys Phe His Asp Ile Val Gly
    210                 215                 220

Ser Ala Tyr Tyr Val Ala Pro Glu Val Leu Lys Arg Arg Ser Gly Pro

225                 230                 235                 240

Glu Ser Asp Val Trp Ser Ile Gly Val Ile Thr Tyr Ile Leu Leu Cys
                245                 250                 255

Gly Arg Arg Pro Phe Trp Asp Lys Thr Glu Asp Gly Ile Phe Lys Glu
            260                 265                 270

Val Leu Arg Asn Lys Pro Asp Phe Arg Lys Arg Pro Trp Ser Ser Ile
        275                 280                 285

Ser Pro Gly Ala Lys Asp Phe Val Lys Arg Leu Leu Val Lys Asn Pro
    290                 295                 300

Arg Ala Arg Leu Thr Ala Ala Gln Ala Leu Ser His Pro Trp Val Arg
305                 310                 315                 320

Glu Gly Gly Glu Ala Ser Asp Ile Pro Val Asp Ile Ser Val Leu Ser
                325                 330                 335

Asn Met Arg Gln Phe Val Lys Tyr Ser Arg Phe Lys Gln Phe Ala Leu
            340                 345                 350

Arg Ala Leu Ala Ser Thr Leu Asn Glu Glu Glu Leu Ser Asp Leu Lys
        355                 360                 365

Asp Gln Phe Asp Ala Ile Asp Ile Asp Lys Ser Gly Ser Ile Ser Ile
    370                 375                 380

Glu Glu Met Arg His Ala Leu Ala Lys Asp Leu Pro Trp Arg Leu Lys
385                 390                 395                 400

Gly Pro Arg Val Leu Glu Ile Ile Gln Ala Ile Asp Ser Asn Thr Asp
                405                 410                 415

Gly Leu Val Asp Phe Lys Glu Phe Val Ala Ala Thr Leu His Ile His
            420                 425                 430

Gln Met Ala Glu Leu Asp Ser Glu Arg Trp Gly Ile Arg Cys Gln Ala
        435                 440                 445

Ala Phe Ser Lys Phe Asp Leu Asp Gly Asp Gly Tyr Ile Thr Pro Glu
    450                 455                 460

Glu Leu Arg Met His Pro Gly Leu Lys Gly Ser Ile Glu Pro Leu Leu
465                 470                 475                 480

Glu Glu Ala Asp Ile Asp Lys Asp Gly Lys Ile Ser Leu Ser Glu Phe
                485                 490                 495

Arg Lys Leu Leu Arg Thr Ala Ser Met Ser Asn Val Pro Ser Pro Arg
            500                 505                 510

Gly Pro Pro Asn Pro Gln Ala Leu
        515                 520

<210>  3
<211>  1934
<212>  DNA
<213>  Zea mays L.

<400>  3
aaatccatat gctcagctcc cgcctcctcc catccccgga ccccggaccc cggccatggg      60

cgcttgcttc tcctccgcct ctgccgcccc cgccggcgcc gccgtcgacg agcgccgccc     120

gtccaaggag ggcgacggca agaagaggcg ccgcgccgcc ggggcatcgc cggatgccgc     180

ggcgcccgtg cgcgtggagt tcggctacga gagggacttc gaggcgcgct acgaggtcgg     240

ccgcctgctc ggccacggcc agttcggcta caccttcgcc gccaccgacc gcggctctgg     300

ggaccgcgtt gccgtcaagc gcatcgacaa ggccaagatg acccgccctg ttgctgtgga     360

ggatgtgaaa agagaagtga agattcttaa agcacttaaa ggacatcaga atattgttca     420

cttctacaat gcatttgagg atgattcata cgtgtacatt gtgatggagc tatgtgaggg     480

cggtgaacta ttagatcgga ttttggcaaa aaagaatagc cgctatagtg agaaagatgc     540

tgcagtggta gtccgccaaa tgctcaaagt agctgctgaa tgccatctgc gtgggttagt     600

tcaccgagat atgaagcctg agaacttcct tttcaaatcg aacaaggagg attcaccact     660

aaaggcgaca gattttggtt tgtcagattt cattaagcca gggaagaagt tccatgacat     720

tgttggaagt gcttactatg tcgcaccaga agtactaaaa cgacggtctg gtcctgagtc     780

agatgtttgg agcataggag tcataaccta cattttgctc tgtgggaggc gccctttttg     840

ggataagacc gaagacggta tattcaagga ggttctaagg aacaagcctg attttcgtaa      900

gaggccttgg tcaagcatca gcccaggtgc taaagatttt gttaaaaggt tactagtgaa     960

gaatccaagg gccaggctaa cagctgctca agctctctca catccgtggg taagagaagg    1020

aggggaagca tccgatatcc ccgtcgacat atctgtgtta tcaaacatgc gtcagtttgt    1080

caagtacagc cgtttcaagc aattcgcgct tcgggctctg gcgagcaccc ttaacgagga    1140

agagctatca gatctgaagg atcagtttga tgcaattgat atcgataaaa gtggatcgat    1200

tagtatcgag gaaatgcgtc atgcccttgc aaaggatctt ccctggagat tgaagggtcc     1260

ccgtgtgctg gagattattc aagcaattga cagcaacact gatgggctcg tggacttcaa    1320

ggagtttgtt gcggcaactc tccatatcca ccagatggcg gagctcgact cagaaaggtg    1380

gggcatacgc tgccaagctg ctttcagtaa gtttgatctt gacggtgatg gatatatcac 1440

gccggaggaa ctcagaatgc accctgggtt gaagggatct atcgagccgc tgctggagga 1500

ggccgacatc gacaaagacg gcaagataag cctgtccgag ttccgcaagc tcctacggac 1560

agcgagcatg agcaacgtac ccagcccaag gggcccccca aaccctcagg ctctgtgaat 1620

tccggctcgg ccactaggga ggagcaagct taggaagttg ccatacaata gccatgtgtt 1680

ctttgggttc ttcagagtgc catgtgatgt ttctggtttt tagcatccag gttatgtgtg 1740

cagtgcagcc ccgagtgagt ttcgaagtaa atattcagtg ctttcttttt ctttccggaa 1800

gagtgagagg tggaggtcaa aatggtaggc aagactcgcc ttcttctttc ctttacactg 1860

tacagtgata ctgaaatatg tacgattttt attataactg ttcgtcgcaa taaagttatt 1920

tggagaagtg agga 1934

<210> 4
<211> 522
<212> PRT
<213> Zea mays L.

<400> 4

Met Gly Ala Cys Phe Ser Ser Ala Ser Ala Ala Pro Ala Gly Ala Ala
1               5                   10                  15

Val Asp Glu Arg Arg Pro Ser Lys Glu Gly Asp Gly Lys Lys Arg Arg
            20                  25                  30

Arg Ala Ala Gly Ala Ser Pro Asp Ala Ala Ala Pro Val Arg Val Glu
        35                  40                  45

Phe Gly Tyr Glu Arg Asp Phe Glu Ala Arg Tyr Glu Val Gly Arg Leu
    50                  55                  60

Leu Gly His Gly Gln Phe Gly Tyr Thr Phe Ala Ala Thr Asp Arg Gly
65                  70                  75                  80

Ser Gly Asp Arg Val Ala Val Lys Arg Ile Asp Lys Ala Lys Met Thr
                85                  90                  95

Arg Pro Val Ala Val Glu Asp Val Lys Arg Glu Val Lys Ile Leu Lys
            100                 105                 110

Ala Leu Lys Gly His Gln Asn Ile Val His Phe Tyr Asn Ala Phe Glu
        115                 120                 125

Asp Asp Ser Tyr Val Tyr Ile Val Met Glu Leu Cys Glu Gly Gly Glu
    130                 135                 140

Leu Leu Asp Arg Ile Leu Ala Lys Lys Asn Ser Arg Tyr Ser Glu Lys
145                 150                 155                 160

Asp Ala Ala Val Val Val Arg Gln Met Leu Lys Val Ala Ala Glu Cys
                165                 170                 175

His Leu Arg Gly Leu Val His Arg Asp Met Lys Pro Glu Asn Phe Leu
            180                 185                 190

Phe Lys Ser Asn Lys Glu Asp Ser Pro Leu Lys Ala Thr Asp Phe Gly
        195                 200                 205

Leu Ser Asp Phe Ile Lys Pro Gly Lys Lys Phe His Asp Ile Val Gly
    210                 215                 220

Ser Ala Tyr Tyr Val Ala Pro Glu Val Leu Lys Arg Arg Ser Gly Pro
225                 230                 235                 240

Glu Ser Asp Val Trp Ser Ile Gly Val Ile Thr Tyr Ile Leu Leu Cys
                245                 250                 255

Gly Arg Arg Pro Phe Trp Asp Lys Thr Glu Asp Gly Ile Phe Lys Glu
            260                 265                 270

Val Leu Arg Asn Lys Pro Asp Phe Arg Lys Arg Pro Trp Ser Ser Ile
        275                 280                 285

Ser Pro Gly Ala Lys Asp Phe Val Lys Arg Leu Leu Val Lys Asn Pro
    290                 295                 300

Arg Ala Arg Leu Thr Ala Ala Gln Ala Leu Ser His Pro Trp Val Arg
305                 310                 315                 320

Glu Gly Gly Glu Ala Ser Asp Ile Pro Val Asp Ile Ser Val Leu Ser
                325                 330                 335

Asn Met Arg Gln Phe Val Lys Tyr Ser Arg Phe Lys Gln Phe Ala Leu
            340                 345                 350

Arg Ala Leu Ala Ser Thr Leu Asn Glu Glu Glu Leu Ser Asp Leu Lys
        355                 360                 365

Asp Gln Phe Asp Ala Ile Asp Ile Asp Lys Ser Gly Ser Ile Ser Ile
    370                 375                 380

Glu Glu Met Arg His Ala Leu Ala Lys Asp Leu Pro Trp Arg Leu Lys

385                 390                 395                 400

Gly Pro Arg Val Leu Glu Ile Ile Gln Ala Ile Asp Ser Asn Thr Asp
                405                 410                 415

Gly Leu Val Asp Phe Lys Glu Phe Val Ala Ala Thr Leu His Ile His
            420                 425                 430

Gln Met Ala Glu Leu Asp Ser Glu Arg Trp Gly Ile Arg Cys Gln Ala
        435                 440                 445

Ala Phe Ser Lys Phe Asp Leu Asp Gly Asp Gly Tyr Ile Thr Pro Glu
    450                 455                 460

Glu Leu Arg Met Val Gln His Pro Gly Leu Lys Gly Ser Ile Glu Pro
465                 470                 475                 480

Leu Leu Glu Glu Ala Asp Ile Asp Lys Asp Gly Lys Ile Ser Leu Ser
                485                 490                 495

Glu Phe Arg Lys Leu Leu Arg Thr Ala Ser Met Ser Asn Val Pro Ser
            500                 505                 510

Pro Arg Gly Pro Pro Asn Pro Gln Ala Leu
        515                 520

<210>  5
<211>  1940
<212>  DNA
<213>  Zea mays L.

<400>  5
aaatccatat gctcagctcc cgcctcctcc catccccgga ccccggaccc cggccatggg      60

cgcttgcttc tcctccgcct ctgccgcccc cgccggcgcc gccgtcgacg agcgccgccc     120

gtccaaggag ggcgacggca agaagaggcg ccgcgccgcc ggggcatcgc cggatgccgc     180

ggcgcccgtg cgcgtggagt tcggctacga gagggacttc gaggcgcgct acgaggtcgg     240

ccgcctgctc ggccacggcc agttcggcta caccttcgcc gccaccgacc gcggctctgg     300

ggaccgcgtt gccgtcaagc gcatcgacaa ggccaagatg acccgccctg ttgctgtgga     360

ggatgtgaaa agagaagtga agattcttaa agcacttaaa ggacatcaga atattgttca     420

cttctacaat gcatttgagg atgattcata cgtgtacatt gtgatggagc tatgtgaggg     480

cggtgaacta ttagatcgga ttttggcaaa aaagaatagc cgctatagtg agaaagatgc     540

tgcagtggta gtccgccaaa tgctcaaagt agctgctgaa tgccatctgc gtgggttagt     600

tcaccgagat atgaagcctg agaacttcct tttcaaatcg aacaaggagg attcaccact     660

aaaggcgaca gattttggtt tgtcagattt cattaagcca gggaagaagt tccatgacat 720

tgttggaagt gcttactatg tcgcaccaga agtactaaaa cgacggtctg gtcctgagtc 780

agatgtttgg agcataggag tcataaccta cattttgctc tgtgggaggc gccctttttg 840

ggataagacc gaagacggta tattcaagga ggttctaagg aacaagcctg attttcgtaa 900

gaggccttgg tcaagcatca gcccaggtgc taaagatttt gttaaaaggt tactagtgaa 960

gaatccaagg gccaggctaa cagctgctca agctctctca catccgtggg taagagaagg 1020

aggggaagca tccgatatcc ccgtcgacat atctgtgtta tcaaacatgc gtcagtttgt 1080

caagtacagc cgtttcaagc aattcgcgct tcgggctctg gcgagcaccc ttaacgagga 1140

agagctatca gatctgaagg atcagtttga tgcaattgat atcgataaaa gtggatcgat 1200

tagtatcgag gaaatgcgtc atgcccttgc aaaggatctt ccctggagat tgaagggtcc 1260

ccgtgtgctg gagattattc aagcaattga cagcaacact gatgggctcg tggacttcaa 1320

ggagtttgtt gcggcaactc tccatatcca ccagatggcg gagctcgact cagaaaggtg 1380

gggcatacgc tgccaagctg ctttcagtaa gtttgatctt gacggtgatg gatatatcac 1440

gccggaggaa ctcagaatgg tgcagcaccc tgggttgaag ggatctatcg agccgctgct 1500

ggaggaggcc gacatcgaca aagacggcaa gataagcctg tccgagttcc gcaagctcct 1560

acggacagcg agcatgagca acgtacccag cccaaggggg cccccaaacc ctcaggctct 1620

gtgaattccg gctcggccac tagggaggag caagcttagg aagttgccat acaatagcca 1680

tgtgttcttt gggttcttca gagtgccatg tgatgtttct ggtttttagc atccaggtta 1740

tgtgtgcagt gcagccccga gtgagtttcg aagtaaatat tcagtgcttt ctttttcttt 1800

ccggaagagt gagaggtgga ggtcaaaatg gtaggcaaga ctcgccttct tctttccttt 1860

acactgtaca gtgatactga aatatgtacg atttttatta taactgttcg tcgcaataaa 1920

gttatttgga gaagtgagga 1940

<210> 6
<211> 6946
<212> DNA
<213> Zea mays L.

<400> 6
gcaaatccat atgctcagct cccgcctcct cccatccccg gaccccggac cccggccatg 60

ggcgcttgct tctcctccgc ctctgccgcc cccgccggcg ccgccgtcga cgagcgccgc 120

ccgtccaagg agggcgacgg caagaagagg cgccgcgccg ccggggcatc gccggatgcc 180

gcggcgcccg tgcgcgtgga gttcggctac gagagggact tcgaggcgcg ctacgaggtc 240

ggccgcctgc tcggccacgg ccagttcggc tacaccttcg ccgccaccga ccgcggctct 300

ggggaccgcg ttgccgtcaa gcgcatcgac aaggccaagg tgagctgccg cctgcccccc 360

cgcaccccaa gccgccgcgc tgtccctgtc tctgtctctc ctactagtag tagtagctgg 420

tggtgattcc gagcgcgtct ttggtctggt gcatcgaacc acttgtgctt ggtgcatttc 480

gaggggattc ggtgtaattc cgtgcaaatt agggatttct ctcctgttgc tttccgaggt 540

ttaggtgttt cgattgggac gcgattggag ccgttcattt taggacattt ccggtgcctt 600

ttgggaggcg tttagctcaa cgagtagctc actcacattt ctagctgttt ggccgcttca 660

tttctcccaa gctttcgttg tttgccggtg gttctgagct gcgggatctt gacgttggcc 720

agagaggtgg tttcgacatt caggcatctc ggatgacctc ttagtttggc actacagctc 780

tattatttcg ggaacgacgt gttgctcagt gcgcacctca ttcatggaag tggcaaggtc 840

gcttgtctgc agaacgggga aggtgctttt catctggcta ttcatggaaa acgacttgtt 900

cagttgccct actaataatt tcaataagat tgcctgcctc cttgaatggt tggggcttgg 960

aaggttcctg tcgaagaaaa agtcaggaaa gataacaatt gcgcacttgc agtggacaac 1020

gcttccctgt cttctatgct ataggtggac agcatttttc taggtataat taatttgacc 1080

ttcaaacata tgtatactaa ccaacgcggt tttgattcca tcaaatgttt tggactctct 1140

ctgctgaact gtcaaagtta cttcatgggg caaaatgtca aattttctgg aaccttccgt 1200

agtatatttt ggaaatgagt gtttattgtg tcattggaaa taccgttcat gtgtctgtga 1260

cagaatgtgt cactagaaag ctgaattggt gttgtccttg tcaaaaaggc actaaacacg 1320

agtctgaaaa ttaggcctgt tcttggtaag ggaaggaatc tgagcatcaa tgctgatagg 1380

aatagactct gtctgtcaat attgttaact tgtttatagg gcttcgagtt ttcaactttt 1440

gaggcagata agtaggatac ctcttttgat catgatatat aacatattct tatatacctc 1500

aagccttgca ctgttaagtt aatgtggcat cctttctaga gatcatgacc tcaagttgca 1560

tatggatgcc aataatatcg acaccaagtg aacatcagtg tctgtggaat atgccgaaag 1620

cagccaacgt gccattactg aattttcata tgattattat attctgttta gatttattta 1680

cgtcggaaca cagtgagatg gtaacgtaat gaatcaaaat aggctataaa catgcaattc 1740

aacatatcat tatcatgccc aagtgttttg tcattctatc tttattcgtc caagaaggac 1800

aagcctggtg cattgttgag ggaaccagtt cttctgcagt acttctaggg aggtaaaaat 1860

tcaacaccgt tggatgcaga tctatcgaac ccaggacttt tgtgcttcca gtgaaaagtt 1920

atatggaccc ataggccaga ggatgtgaga gttttacctc tctggaagtt atatgcgcta 1980

gcattagtgt ggtcatcaat gggatcaaag atgagctcca cctttggtgt agagctggag 2040

ctaggggact ctagcatcct ggcgctccaa tcttccatcc agtgaactct gttttttggg 2100

tctagtaggt caagggtcca gttatttttt ctttctgctg taaagtctct agttaaggtg 2160

tgagttttgt atggtgtttt ttcgaggttt ccccaaacct cacctttttt ccttcttaat 2220

ataatgatat gcagctttcc tgcgtattcg agaaaagaaa gttttatctc tctggaagtt 2280

aactgcagag gaacttgtta cattgttgag agttgtctca ccgagtcacc aggtcgctgg 2340

ttcaaagcag tctctccaca tttatgggga aggcttgcct cggtttatcc cttcccaaga 2400

ctctacttgt gggagactct ggcattgggt ctgtcctatg ccgttgaagc gctaggttcg 2460

tttatccctt ccctataccc acttgtcaga gcctccaaca ctgagtctgc cctaagcttc 2520

caagttccaa cactgggtct gccctaggcc gtcgaagcgt tatatgattg ccatgtactg 2580

ttatgctttg ttgccttcac atattttccg ttcgaaatca tctccttgtt gccttcacat 2640

attgccttgt tgctttcaca tattttctgt tccacgtcat acttagaagt tagaacacgt 2700

gatttatgcc aattaagatt attattttat ataacagatg acccgccctg ttgctgtgga 2760

ggatgtgaaa agagaagtga agattcttaa agcacttaaa ggacatcaga atattgttca 2820

cttctacaat gcatttgagg atgattcata cgtgtacatt gtgatggagt aagtaggccc 2880

atacacctgt tcctgctaat agagcatatc gattttgcta tgactttttt ccctaaagtt 2940

ttaacatgaa caatatctat cctgtttaca gaatcctaga cactaaaatg tcatttctaa 3000

ttatcaatta ttctatagct aaaccagatg caatcctgat ttatttttct taacgtatgg 3060

atatattgga cttttctttc aaacctgcat tttgaatttg attacaggga actataacac 3120

taattcagaa ctctatcatg tttaacattt ttcttgcatt gttctatgtt tgtcaacttg 3180

acgcacttct tagataatat aacatcatct tccacagtca ccattagtta ggaccttgga 3240

ccttcatggt tccgaaattt agctaagaat ggtatacatg gtcatgtgat ttcaaataga 3300

tgttcctata tgccagaacc aactcatgag tcataagttt taccttgtgt ttttgcaggc 3360

tatgtgaggg cggtgaacta ttagatcgga ttttggcaaa gtaagtagat aagatcccca 3420

tctctttgtt tcccgtacct cattcttcgc cattaaattt atagattttt gtgctgtaaa 3480

atcagattgc tttatgttgt ttgtctgctt tgtttgattt ctagttgctc gttcaagatc 3540

ctttacttaa tggtgtgcgt gttttgacag aaagaatagc cgctatagtg agaaagatgc 3600

tgcagtggta gtccgccaaa tgctcaaagt agctgctgaa tgccatctgc gtgggttagt 3660

tcaccgagat atgaagcctg aggtagaaat caaatacttc aatctctttg cacacagtaa 3720

gcatttggtg atatttcact acttcctcag gtcatgtaag actgtaccta ttttccttcc 3780

cagaacttcc ttttcaaatc gaacaaggag gattcaccac taaaggcgac agattttggt 3840

ttgtcagatt tcattaagcc aggtatctac ttggggccat ctgaatctgt cgggaatctg 3900

ataggggcaa gtctgcagtt tagctgacca ttttgttgtc taatgcatgc tttagggaag 3960

aagttccatg acattgttgg aagtgcttac tatgtcgcac cagaagtact aaaacgacgg 4020

tctggtcctg agtcagatgt ttggagcata ggagtcataa cctacatttt gctctgtggg 4080

aggcgccctt tttgggataa gaccgaagac ggtatattca aggaggtaag tggatggatt 4140

ttgcatacca tgtgcttaca tgtaaaatat gcttggttag agtgctgtac cagggatcag 4200

cgttttcagc gtgctgatac tgttttgtac aatgtgtttc tactttctac gtcatatagc 4260

agtgtttctt tgttaactat ttcagtgtca aactatttgt cgtgtcacaa ctcagcagta 4320

taattttact attttgaaca ctgtaaacct gcctggtcag gttatccttc agtaatttct 4380

ctactagcta ccagaaaccc actttatgca ggtgttcagt ttaataacac ccaccatctt 4440

tcagatttct aatgttcagt gttagacaga cttcattaag atgcacctta agatgattgt 4500

aagtagtaaa agtgctttgc acttttgtta acttttgagt ctgaagatga cttgtggtac 4560

ctatgacctc aagaaaccaa ggcattgcca ttggaatagc taattcgaat gagcttcaga 4620

tatggctatc tgttttagtt ttggacatct gactcaactt tataggataa tactatatta 4680

gcaatctttg aggtcattgt ctcagccaaa ataagttgcg gtctcttttt tactgtccta 4740

agcagcaata tggtttccat tttcattata ccagcaactt ccaccttttt cttgctattt 4800

aaatatcttt atgcatttta tcagcaagga catgatacga tcgtatatgt gatattctac 4860

atcttttcac ttctcataat taggttctaa ggaacaagcc tgattttcgt aagaggcctt 4920

ggtcaagcat cagcccaggt gctaaagatt ttgttaaaag gttactagtg aagaatccaa 4980

gggccaggct aacagctgct caagctctct gtaagttttg gtatttttca ttaatttact 5040

agcctagtca tgatgatcag attcaccttc tctatgtgag aacagagaac acatatacat 5100

ctggcagtat gcctttcaat cagttatgac aatgtaaata tgcaaagacc gatgtttttt 5160

ctatcctgca ccattttaga acattaatgg ggaaaaacca caatatatta ggaaaaatgt 5220

ttaattatgt cctggtcact tgaaatgaac atataccact gaggttttct agttctcatg 5280

cgttcttata atgatctaat aagtcagtgg aggtttgctg cccaccaccc ctacatttgt 5340

attgtgaatt actatcatct ttactgatcc tgattgttct tgatatgtta agcacatccg 5400

tgggtaagag aaggagggga agcatccgat atccccgtcg acatatctgt gttatcaaac 5460

atgcgtcagt ttgtcaagta cagccgtttc aagcaattcg cgcttcgggt aattacagtg 5520

attacaaaaa acaacactgc atcgtttatt ttttcctcac aatatttcct cgtggcatgg 5580

tcaggctctg gcgagcaccc ttaacgagga agagctatca gatctgaagg atcagtttga 5640

tgcaattgat atcgataaaa gtggatcgat tagtatcgag gaaatgcgtc atgtaggttc 5700

tgttagtgtt tgctgatgaa aatgccttag atcctgaact actctgcggt gctgattaat 5760

ctgtgcatgt ttcggtaggc ccttgcaaag gatcttccct ggagattgaa gggtccccgt 5820

gtgctggaga ttattcaagc agtaagtttg agccttcttc tggatccagc cctttctttg 5880

ttacccccct tgtttccaag aaaatagctg gccttgttct gagggtataa ccaaaactgc 5940

atcttatttt gtggtagatt gacagcaaca ctgatgggct cgtggacttc aaggagtttg 6000

ttgcggcaac tctccatatc caccagatgg cggagctcga ctcagaaagg tggggcatac 6060

gctgccaagc tgctttcagt aagtttgatc ttgacggtga tggatatatc acgccggagg 6120

aactcagaat ggtaattttc tactcctgtc ttgtttccat gttgcttcac caacgaatgc 6180

acagttcaca taacccttat tatcatcact gcttcccatg aataactagc tggctcgacc 6240

atcatgagat tcagtacttg cgccctgtgc acttggtttt ggtcccgctt gttagaatga 6300

agtaatttat caatggaagc gctgtaatat tttaatcagc gtttagattt gataaagata 6360

aaacatgttc attgtttgtg ccaagaaatc cacttacaca gatactgaga gttgcaccgt 6420

agataacgct aatcggcagt atcctaatcg agatttcttt tcaaggtgca gcacactggg 6480

ttgaagggat ctatcgagcc gctgctggag gaggccgaca tcgacaaaga cggcaagata 6540

agcctgtccg agttccgcaa gctcctacgg acagcgagca tgagcaacgt acccagccca 6600

agggggcccc caaaccctca ggctctgtga attccggctc ggccactagg gaggagcaag 6660

cttaggaagt tgccatacaa tagccatgtg ttctttgggt tcttcagagt gccatgtgat 6720

gtttctggtt tttagcatcc aggttatgtg tgcagtgcag ccccgagtga gtttcgaagt 6780

aaatattcag tgctttcttt ttctttccgg aagagtgaga ggtggaggtc aaaatggtag 6840

gcaagactcg ccttcttctt tcctttacac tgtacagtga tactgaaata tgtacgattt 6900

ttattataac tgttcgtcgc aataaagtta tttggagaag tgagga 6946

<210> 7
<211> 520
<212> PRT
<213> Zea mays L.

<400> 7

Met Gly Ala Cys Phe Ser Ser Ala Ser Ala Ala Pro Ala Gly Ala Ala
1               5                   10                  15

Val Asp Glu Arg Arg Pro Ser Lys Glu Gly Asp Gly Lys Lys Arg Arg
            20                  25                  30

Arg Ala Ala Gly Ala Ser Pro Asp Ala Ala Ala Pro Val Arg Val Glu
        35                  40                  45

Phe Gly Tyr Glu Arg Asp Phe Glu Ala Arg Tyr Glu Val Gly Arg Leu
    50                  55                  60

Leu Gly His Gly Gln Phe Gly Tyr Thr Phe Ala Ala Thr Asp Arg Gly
65                  70                  75                  80

Ser Gly Asp Arg Val Ala Val Lys Arg Ile Asp Lys Ala Lys Met Thr
                85                  90                  95

Arg Pro Val Ala Val Glu Asp Val Lys Arg Glu Val Lys Ile Leu Lys
        100                 105                 110

Ala Leu Lys Gly His Gln Asn Ile Val His Phe Tyr Asn Ala Phe Glu
        115                 120                 125

Asp Asp Ser Tyr Val Tyr Ile Val Met Glu Leu Cys Glu Gly Gly Glu
    130                 135                 140

Leu Leu Asp Arg Ile Leu Ala Lys Lys Asn Ser Arg Tyr Ser Glu Lys
145                 150                 155                 160

Asp Ala Ala Val Val Val Arg Gln Met Leu Lys Val Ala Ala Glu Cys
                165                 170                 175

His Leu Arg Gly Leu Val His Arg Asp Met Lys Pro Glu Asn Phe Leu
            180                 185                 190

Phe Lys Ser Asn Lys Glu Asp Ser Pro Leu Lys Ala Thr Asp Phe Gly
        195                 200                 205

Leu Ser Asp Phe Ile Lys Pro Gly Lys Lys Phe His Asp Ile Val Gly
    210                 215                 220

Ser Ala Tyr Tyr Val Ala Pro Glu Val Leu Lys Arg Arg Ser Gly Pro
225                 230                 235                 240

Glu Ser Asp Val Trp Ser Ile Gly Val Ile Thr Tyr Ile Leu Leu Cys
                245                 250                 255

Gly Arg Arg Pro Phe Trp Asp Lys Thr Glu Asp Gly Ile Phe Lys Glu
            260                 265                 270

Val Leu Arg Asn Lys Pro Asp Phe Arg Lys Arg Pro Trp Ser Ser Ile
        275                 280                 285

Ser Pro Gly Ala Lys Asp Phe Val Lys Arg Leu Leu Val Lys Asn Pro
    290                 295                 300

Arg Ala Arg Leu Thr Ala Ala Gln Ala Leu Ser His Pro Trp Val Arg
305                 310                 315                 320

Glu Gly Gly Glu Ala Ser Asp Ile Pro Val Asp Ile Ser Val Leu Ser
                325                 330                 335

Asn Met Arg Gln Phe Val Lys Tyr Ser Arg Phe Lys Gln Phe Ala Leu
            340                 345                 350

Arg Ala Leu Ala Ser Thr Leu Asn Glu Glu Glu Leu Ser Asp Leu Lys
        355                 360                 365

Asp Gln Phe Asp Ala Ile Asp Ile Asp Lys Ser Gly Ser Ile Ser Ile
    370                 375                 380

Glu Glu Met Arg His Ala Leu Ala Lys Asp Leu Pro Trp Arg Leu Lys
385                 390                 395                 400

Gly Pro Arg Val Leu Glu Ile Ile Gln Ala Ile Asp Ser Asn Thr Asp
                405                 410                 415

Gly Leu Val Asp Phe Lys Glu Phe Val Ala Ala Thr Leu His Ile His
            420                 425                 430

Gln Met Ala Glu Leu Asp Ser Glu Arg Trp Gly Ile Arg Cys Gln Ala
        435                 440                 445

Ala Phe Ser Lys Phe Asp Leu Asp Gly Asp Gly Tyr Ile Thr Pro Glu
    450                 455                 460

Glu Leu Arg Met His Thr Gly Leu Lys Gly Ser Ile Glu Pro Leu Leu
465                 470                 475                 480

Glu Glu Ala Asp Ile Asp Lys Asp Gly Lys Ile Ser Leu Ser Glu Phe
                485                 490                 495

Arg Lys Leu Leu Arg Thr Ala Ser Met Ser Asn Val Pro Ser Pro Arg
            500                 505                 510

Gly Pro Pro Asn Pro Gln Ala Leu
        515                 520

<210>  8
<211>  1936
<212>  DNA
<213>  Zea mays L.

<400>  8
aaatccatat gctcagctcc cgcctcctcc catccccgga ccccggaccc cggccatggg      60

cgcttgcttc tcctccgcct ctgccgcccc cgccggcgcc gccgtcgacg agcgccgccc     120

gtccaaggag ggcgacggca agaagaggcg ccgcgccgcc ggggcatcgc cggatgccgc     180

ggcgcccgtg cgcgtggagt tcggctacga gagggacttc gaggcgcgct acgaggtcgg     240

ccgcctgctc ggccacggcc agttcggcta caccttcgcc gccaccgacc gcggctctgg     300

ggaccgcgtt gccgtcaagc gcatcgacaa ggccaagatg acccgccctg ttgctgtgga     360

ggatgtgaaa agagaagtga agattcttaa agcacttaaa ggacatcaga atattgttca 420

cttctacaat gcatttgagg atgattcata cgtgtacatt gtgatggagc tatgtgaggg 480

cggtgaacta ttagatcgga ttttggcaaa aaagaatagc cgctatagtg agaaagatgc 540

tgcagtggta gtccgccaaa tgctcaaagt agctgctgaa tgccatctgc gtgggttagt 600

tcaccgagat atgaagcctg agaacttcct tttcaaatcg aacaaggagg attcaccact 660

aaaggcgaca gattttggtt tgtcagattt cattaagcca gggaagaagt tccatgacat 720

tgttggaagt gcttactatg tcgcaccaga agtactaaaa cgacggtctg gtcctgagtc 780

agatgtttgg agcataggag tcataaccta cattttgctc tgtgggaggc gccctttttg 840

ggataagacc gaagacggta tattcaagga ggttctaagg aacaagcctg attttcgtaa 900

gaggccttgg tcaagcatca gcccaggtgc taaagatttt gttaaaaggt tactagtgaa 960

gaatccaagg gccaggctaa cagctgctca agctctctca catccgtggg taagagaagg 1020

aggggaagca tccgatatcc ccgtcgacat atctgtgtta tcaaacatgc gtcagtttgt 1080

caagtacagc cgtttcaagc aattcgcgct tcgggctctg gcgagcaccc ttaacgagga 1140

agagctatca gatctgaagg atcagtttga tgcaattgat atcgataaaa gtggatcgat 1200

tagtatcgag gaaatgcgtc atgcccttgc aaaggatctt ccctggagat tgaagggtcc 1260

ccgtgtgctg gagattattc aagcaattga cagcaacact gatgggctcg tggacttcaa 1320

ggagtttgtt gcggcaactc tccatatcca ccagatggcg gagctcgact cagaaaggtg 1380

gggcatacgc tgccaagctg ctttcagtaa gtttgatctt gacggtgatg gatatatcac 1440

gccggaggaa ctcagaatgc acactgggtt gaagggatct atcgagccgc tgctggagga 1500

ggccgacatc gacaaagacg gcaagataag cctgtccgag ttccgcaagc tcctacggac 1560

agcgagcatg agcaacgtac ccagcccaag gggcccccca aaccctcagg ctctgtgaat 1620

tccggctcgg ccactaggga ggagcaagct taggaagttg ccatacaata gccatgtgtt 1680

ctttgggttc ttcagagtgc catgtgatgt ttctggtttt tagcatccag gttatgtgtg 1740

cagtgcagcc ccgagtgagt ttcgaagtaa atattcagtg ctttcttttt ctttccggaa 1800

gagtgagagg tggaggtcaa aatggtaggc aagactcgcc ttcttctttc ctttacactg 1860

tacagtgata ctgaaatatg tacgattttt attataactg ttcgtcgcaa taaagttatt 1920

tggagaagtg aggatt 1936

<210> 9
<211> 522
<212> PRT
<213> Zea mays L.

<400> 9

Met Gly Ala Cys Phe Ser Ser Ala Ser Ala Ala Pro Ala Gly Ala Ala

1               5                   10                  15

Val Asp Glu Arg Arg Pro Ser Lys Glu Gly Asp Gly Lys Lys Arg Arg
            20                  25                  30

Arg Ala Ala Gly Ala Ser Pro Asp Ala Ala Ala Pro Val Arg Val Glu
        35                  40                  45

Phe Gly Tyr Glu Arg Asp Phe Glu Ala Arg Tyr Glu Val Gly Arg Leu
    50                  55                  60

Leu Gly His Gly Gln Phe Gly Tyr Thr Phe Ala Ala Thr Asp Arg Gly
65                  70                  75                  80

Ser Gly Asp Arg Val Ala Val Lys Arg Ile Asp Lys Ala Lys Met Thr
                85                  90                  95

Arg Pro Val Ala Val Glu Asp Val Lys Arg Glu Val Lys Ile Leu Lys
            100                 105                 110

Ala Leu Lys Gly His Gln Asn Ile Val His Phe Tyr Asn Ala Phe Glu
        115                 120                 125

Asp Asp Ser Tyr Val Tyr Ile Val Met Glu Leu Cys Glu Gly Gly Glu
    130                 135                 140

Leu Leu Asp Arg Ile Leu Ala Lys Lys Asn Ser Arg Tyr Ser Glu Lys
145                 150                 155                 160

Asp Ala Ala Val Val Val Arg Gln Met Leu Lys Val Ala Ala Glu Cys
                165                 170                 175

His Leu Arg Gly Leu Val His Arg Asp Met Lys Pro Glu Asn Phe Leu
            180                 185                 190

Phe Lys Ser Asn Lys Glu Asp Ser Pro Leu Lys Ala Thr Asp Phe Gly
        195                 200                 205

Leu Ser Asp Phe Ile Lys Pro Gly Lys Lys Phe His Asp Ile Val Gly
    210                 215                 220

Ser Ala Tyr Tyr Val Ala Pro Glu Val Leu Lys Arg Arg Ser Gly Pro
225                 230                 235                 240

Glu Ser Asp Val Trp Ser Ile Gly Val Ile Thr Tyr Ile Leu Leu Cys
                245                 250                 255

Gly Arg Arg Pro Phe Trp Asp Lys Thr Glu Asp Gly Ile Phe Lys Glu
            260                 265                 270

Val Leu Arg Asn Lys Pro Asp Phe Arg Lys Arg Pro Trp Ser Ser Ile
        275                 280                 285

Ser Pro Gly Ala Lys Asp Phe Val Lys Arg Leu Leu Val Lys Asn Pro
    290                 295                 300

Arg Ala Arg Leu Thr Ala Ala Gln Ala Leu Ser His Pro Trp Val Arg
305                 310                 315                 320

Glu Gly Gly Glu Ala Ser Asp Ile Pro Val Asp Ile Ser Val Leu Ser
                325                 330                 335

Asn Met Arg Gln Phe Val Lys Tyr Ser Arg Phe Lys Gln Phe Ala Leu
            340                 345                 350

Arg Ala Leu Ala Ser Thr Leu Asn Glu Glu Glu Leu Ser Asp Leu Lys
        355                 360                 365

Asp Gln Phe Asp Ala Ile Asp Ile Asp Lys Ser Gly Ser Ile Ser Ile
    370                 375                 380

Glu Glu Met Arg His Ala Leu Ala Lys Asp Leu Pro Trp Arg Leu Lys
385                 390                 395                 400

Gly Pro Arg Val Leu Glu Ile Ile Gln Ala Ile Asp Ser Asn Thr Asp
                405                 410                 415

Gly Leu Val Asp Phe Lys Glu Phe Val Ala Ala Thr Leu His Ile His
            420                 425                 430

Gln Met Ala Glu Leu Asp Ser Glu Arg Trp Gly Ile Arg Cys Gln Ala
        435                 440                 445

Ala Phe Ser Lys Phe Asp Leu Asp Gly Asp Gly Tyr Ile Thr Pro Glu
    450                 455                 460

Glu Leu Arg Met Val Gln His Thr Gly Leu Lys Gly Ser Ile Glu Pro
465                 470                 475                 480

Leu Leu Glu Glu Ala Asp Ile Asp Lys Asp Gly Lys Ile Ser Leu Ser
                485                 490                 495

Glu Phe Arg Lys Leu Leu Arg Thr Ala Ser Met Ser Asn Val Pro Ser
            500                 505                 510

Pro Arg Gly Pro Pro Asn Pro Gln Ala Leu
        515                 520

<210>  10
<211>  1940
<212>  DNA
<213>  Zea mays L.

<400>  10
aaatccatat gctcagctcc cgcctcctcc catccccgga ccccggaccc cggccatggg      60

cgcttgcttc tcctccgcct ctgccgcccc cgccggcgcc gccgtcgacg agcgccgccc     120

gtccaaggag ggcgacggca agaagaggcg ccgcgccgcc ggggcatcgc cggatgccgc     180

ggcgcccgtg cgcgtggagt tcggctacga gagggacttc gaggcgcgct acgaggtcgg     240

ccgcctgctc ggccacggcc agttcggcta caccttcgcc gccaccgacc gcggctctgg     300

ggaccgcgtt gccgtcaagc gcatcgacaa ggccaagatg acccgccctg ttgctgtgga     360

ggatgtgaaa agagaagtga agattcttaa agcacttaaa ggacatcaga atattgttca     420

cttctacaat gcatttgagg atgattcata cgtgtacatt gtgatggagc tatgtgaggg     480

cggtgaacta ttagatcgga ttttggcaaa aaagaatagc cgctatagtg agaaagatgc     540

tgcagtggta gtccgccaaa tgctcaaagt agctgctgaa tgccatctgc gtgggttagt     600

tcaccgagat atgaagcctg agaacttcct tttcaaatcg aacaaggagg attcaccact     660

aaaggcgaca gattttggtt tgtcagattt cattaagcca gggaagaagt tccatgacat     720

tgttggaagt gcttactatg tcgcaccaga agtactaaaa cgacggtctg gtcctgagtc     780

agatgtttgg agcataggag tcataaccta cattttgctc tgtgggaggc gccctttttg     840

ggataagacc gaagacggta tattcaagga ggttctaagg aacaagcctg attttcgtaa     900

gaggccttgg tcaagcatca gcccaggtgc taaagatttt gttaaaaggt tactagtgaa     960

gaatccaagg gccaggctaa cagctgctca agctctctca catccgtggg taagagaagg    1020

aggggaagca tccgatatcc ccgtcgacat atctgtgtta tcaaacatgc gtcagtttgt    1080

caagtacagc cgtttcaagc aattcgcgct tcgggctctg gcgagcaccc ttaacgagga    1140

agagctatca gatctgaagg atcagtttga tgcaattgat atcgataaaa gtggatcgat    1200

tagtatcgag gaaatgcgtc atgcccttgc aaaggatctt ccctggagat tgaagggtcc    1260

ccgtgtgctg gagattattc aagcaattga cagcaacact gatgggctcg tggacttcaa    1320

ggagtttgtt gcggcaactc tccatatcca ccagatggcg gagctcgact cagaaaggtg    1380

gggcatacgc tgccaagctg ctttcagtaa gtttgatctt gacggtgatg gatatatcac    1440

gccggaggaa ctcagaatgg tgcagcacac tgggttgaag ggatctatcg agccgctgct    1500

ggaggaggcc gacatcgaca aagacggcaa gataagcctg tccgagttcc gcaagctcct    1560

acggacagcg agcatgagca acgtacccag cccaaggggg cccccaaacc ctcaggctct 1620

gtgaattccg gctcggccac tagggaggag caagcttagg aagttgccat acaatagcca 1680

tgtgttcttt gggttcttca gagtgccatg tgatgtttct ggtttttagc atccaggtta 1740

tgtgtgcagt gcagccccga gtgagtttcg aagtaaatat tcagtgcttt ctttttcttt 1800

ccggaagagt gagaggtgga ggtcaaaatg gtaggcaaga ctcgccttct tctttccttt 1860

acactgtaca gtgatactga aatatgtacg atttttatta taactgttcg tcgcaataaa 1920

gttatttgga gaagtgagga 1940

<210> 11
<211> 287
<212> DNA
<213> Zea mays L.

<400> 11
tcaccgagat atgaagcctg agaacttcct tttcaaatcg aacaaggagg attcaccact 60

aaaggcgaca gattttggtt tgtcagattt cattaagcca gggaagaagt tccatgacat 120

tgttggaagt gcttactatg tcgcaccaga agtactaaaa cgacggtctg gtcctgagtc 180

agatgtttgg agcataggag tcataaccta cattttgctc tgtgggaggc gccctttttg 240

ggataagacc gaagacggta tattcaagga ggttctaagg aacaagc 287

<210> 12
<211> 13563
<212> DNA
<213> Zea mays L.

<400> 12
gggatctgca gctgcttgaa tgcagcatgg ctgatgacgt tgagcccagc cccaccgtca 60

atcagaacat ggtgcagccg catgttggtg atgacaaggg cgatgatgag cggtaatata 120

ccggcccctg ccatgttgtc ggggcagtcg gatgccccaa aagagatagt ggtgctcctc 180

caccgttgat gtggagcagc catcgggacc cccgggaccg ccgaaaggac ctctcggcac 240

agggacttga tgttcctgcg ggaggtgagc tcccagctcc cgtcgtatat catgtacaac 300

ttcttgcggc ggtcgttgtc atcaccggag tcggagtctc cagtgaaaac atccttcagg 360

accccctcag gagactgata tctgaggtcc cgttctcccg cgaccacatc accgttgtcg 420

accctctcct tgccaggccg gcgacgaggt ggggagccat ccttggaagt ctgctcgcac 480

cgctcgctga tgcgcttcgc gagcttgatg atctcgcggc actccgaggc actgtggcga 540

ctgttggggt gcacagggca tgagccgctg ttgcctccct gtggccgtgg gcgcttgttg 600

cgctcgttcc ggcccccagt tgcggctgcg acgatcctag cagtagactg cggcctctcg 660

tggccgcggt tcttcttctt cttctttttg ccgtcctggg tgacgacacc cgagccaccc 720

gtcttagcaa ctccggtttg tggtgtcgag tgccatgcac ggccctcggc agctctggca 780

cacttgtcag ctagagcgaa gagcgtggtg aaagtttcca cgtcatgcgt ggccaacttc 840

tccagcatct tctcatcacg tactctctgg cggaaagcgg tgatgatgga agcatcgaag 900

atacgaggta tagcgcctcg taccttggtg aagcgggaga tgaaggcccg gagagtttcc 960

ccgggttcct gcctcactgc atggaggtga gcctccatgc catgctgttg ataagcactg 1020

gcgaagttcg ctatgaacca cgcgcaaagc tcttcccagg agtagatcga tcctagggtg 1080

agattcatga gccaggtctg ggctggccca gacaaggcta catggaaata tgttgccatt 1140

acgacagtgt ctccacctgc tgccgtaata gcggtacgta tacctgcaag aattccgaca 1200

ggtttgacgt accgtcgtat tttttggcag gtgtggccgg aacttggatg gccaagtcgc 1260

cgcgctgaga tgatccgcta gtgcggcgca gcccacaccg gtcaatggaa cactcgcctg 1320

aattcgggcg cccgttggag tttgcggtgc aaccgcagcg aggtctaggt cgaggttgcg 1380

accctcgatg ttctaccggc gctctcgcgc cctctccaga gagactcggg catcctcgcc 1440

cgtacgccta cggttgagtt ctgctcgcag gtcatctggc ctctctagag agactggagc 1500

gtcctctccc gcacgcctgc ggttgagctc cgcccgcagg tcctcagtcg gtgcggcctt 1560

caccgaggtt gagcgcactg acgtcgacgc ctcgtgttga cgccgggatg accgaggcct 1620

aggcctcgtt gagccagaat gcgccatact gagcagacga tcgacatcat cacaccactg 1680

cttcatggcc tccggtgagg ccgtggagct aggagggtgg cgtagcaact ccctggctgc 1740

agacaaagcc ccaagtgcag cccttgacgc tctggatgtc tgcgcaggag tgtgctgccg 1800

cgcagagcgc atagcagcag caccaggcgc atggcggttg gaggcccgtg gcatggaaga 1860

cgcagcttct tcctccatat ggaagtcctc aggaacgaag tcgtggtgct cgacgatctg 1920

gaccatggtg tcgagaagga aaacaggcga aaacctaatg ccaagcccct acctggcgcg 1980

ccaaatgttg gagaggaaaa tctccgggcg ggtggcggaa cgcacccgcc ctaaatccta 2040

agatgaggag ggggcttaag cgtattgcct gtctattaga tggtcgatga acacgagagc 2100

acacaaggat ttagagtggt tcaggccgct ggagcgtaat accctactcc actgtttgtg 2160

tgatgtattg agtctgtgag cttgagagag ctcgtgagtc tgagttgggt ctgccttgta 2220

acgttgtgtg ccctcccttt tatagctcaa ggggggcaca tacaaggatg ttgagccccg 2280

acacgtgggc ccagtagcat aatgaaagaa atacattata ggagtaacta atgcaagtaa 2340

cgcataagca atctccggtc gtcgtgatgt ccgcagtcta tgcagtattg atatgcggcg 2400

gctgcttcct tggtaacgtg cgagtaatga tgagcgtagt gcacgcggca gtatgggcgt 2460

gccgcctgcc agtggaatgg acatttcgcc gcctgccagc ggaatggata ggtctcaata 2520

aatgcagagg cggcacaccg tctgccagtg gaatggacag gcgacgcgcc ttatccacaa 2580

taaatgcaga ggtcgcgtag cccagaggcc ttacgtcagg cttcacccgt tggcttacgt 2640

cacgtgcagt gtgccacgtg gcagcatcgg ggctctgcct gggcggggag caggagtgta 2700

tgcagacagg tccggacctg cacacgtgtc agcaccggac ctcgcttggg tcttgttcca 2760

ggctcgagta tgttctgtcc tagaacctta ggaccccatt gtgggcgatc cggacccccc 2820

cacggggggg ggggaggggg gtacagatcc cattctaggg gtcctccttg cacacgtgga 2880

ggtcctggac caaccttgga ggtccggact gtttatcaag gggtccggcg ctctcctatg 2940

ggggtccgga cttactgttg atgccttaga gtatatcacc ttctctagac acgtggcggc 3000

tccggacccg cccatgtggt ggatccgggc gctgctgtgg acccagagta gtcgcccgag 3060

actggggcaa gtcgtggcct cgtcccacac acagcacctt taccacgcga ctaagagata 3120

gccgcgtggg cactgcgtct ttatacaata gtagggggta cccctgtttc agggtactga 3180

caatatcttt tttggatttt tttatgcgta ttttgtccgc tcacctaaac atttttaggt 3240

tatgtttaga tatacagtga aagttaaaca tctgaaaaag ataacccaca atgttaagta 3300

gatggagtac tatagtgaaa caaataaata gtcaaagtta atttagaata acaaactcat 3360

acaaatctta aaatgtcatt ttgtttgtga ctatagaaag tattcttaga gctaggaggt 3420

gcaataaata agacgggcaa gtcaacaacc tcttcgcttg gctttaattt ttaccagctt 3480

ctactatttt tataatattt tatctctata gaatacaact acatcagttt gaatatctag 3540

ctttaattcg aggcgaacaa tctgaaaaac cttatgctta taagggtagc atcaatggta 3600

taggcgtgta tatagcaacg ttcatgttag ctacatatta tactatagca aagtaatgat 3660

atatctatgg tagatattta taaatgtttt taatgattaa atacactctc tgtaccacct 3720

cggtttctgt gtataagttt actccatgtt catagataac agcacactct cacttcatta 3780

atttcttgcc acatatgata gataccgaga gcatctccaa tagactagcc aaatagaccg 3840

tttagccaaa ttttggctaa tcaatagcaa aataactctc caacagacta gccatctgac 3900

tcgtcaaact tttcgactct tcaaattggc tccctcacta gacaaacctg gctagtcact 3960

ttgactaacc aaactagata gatagtatgc tggagtgagt tgctatatac agagtgtaat 4020

atttatgaaa aaaataaata gagagtcaaa taaaaagcaa aaatggagat cccttagaga 4080

tgttttgaca ttggttcttt atagagagct aattgggtac cgttgcggct gccctaattg 4140

accaagactg aggcaagcaa cactagtcac tgatgattag gagatgcttg aatgcactaa 4200

agctaatagt tagttgacta aaaattacta ataaaattaa ctagccaaca aatagctagc 4260

taactagttg ctaatttatt aaaagtagct aataactgaa ctattagtta gactgtttgg 4320

atgtctcagc taattttaac agctaactat tagttctagt gtattaaaac acttccaaat 4380

aatatgcaag tatttaagat aagatcgatg acaacaacat gacgaaataa atcaacctat 4440

caaccaaatt gccaggcatc acatgtatct tgtgtggcta accaaaagga acgacccggc 4500

ggtggaggta gagctgacca agacagcgct gttgccatgg agacatttct tttagtggtc 4560

ttgtttaaac agtaatattt gtatagtaaa tgttgttaga taaattattg tgaattctat 4620

tttcgtaata tttacttaga tgtgtcatgt gtgtggagtg accactgaga ggtgtttgac 4680

aattggcagt ttgaccgctt gaccagcggc ttcatcatca catcgtctcg tctgtcgtcg 4740

tgtgtggctt cctctcttcc cctcctccct tggccccgtc tcgtctcctc cccctcacct 4800

tcctctccac cttcctccct cccacccctc cgcgcctcgc cccaccgcgc caaccaacca 4860

acacggcgtc ccagcctgcc tatataccgc tccccccgcg cccccacacg cgcaaatcca 4920

tatgctcagc tcccgcctcc tcccatcccc ggaccccgga ccccggccat gggcgcttgc 4980

ttctcctccg cctctgccgc ccccgccggc gccgccgtcg acgagcgccg cccgtccaag 5040

gagggcgacg gcaagaagag gcgccgcgcc gccggggcat cgccggatgc cgcggcgccc 5100

gtgcgcgtgg agttcggcta cgagagggac ttcgaggcgc gctacgaggt cggccgcctg 5160

ctcggccacg gccagttcgg ctacaccttc gccgccaccg accgcggctc tggggaccgc 5220

gttgccgtca agcgcatcga caaggccaag gtgagctgcc gcctgccccc ccgcacccca 5280

agccgccgcg ctgtccctgt ctctgtctct cctactagta gtagtagctg gtggtgattc 5340

cgagcgcgtc tttggtctgg tgcatcgaac cacttgtgct tggtgcattt cgaggggatt 5400

cggtgtaatt ccgtgcaaat tggggatttc tctcctgttg ctttccgagg tttaggtgtt 5460

tcgattggga cgcgattgga gccgttcatt ttaggacatt tccggtgcct tttgggaggc 5520

gtttagctca acgagtagct cactcacatt tctagctgtt tggccgcttc atttctccca 5580

agctttcgtt gtttgccggt ggttctgagc tgcgggatct tgacgttggc cagagaggtg 5640

gtttcgacat tcaggcatct cggatgacct cttagtttgg cactacagct ctattatttc 5700

gggaacgacg tgttgctcag tgcgcacctc attcatggaa gtggcaaggt cgcttgtctg 5760

cagaacgggg aaggtgcttt tcatctggct attcatggaa aacgacttgt tcagttgccc 5820

tactaataat ttcaataaga ttgcctgcct ccttgaatgg ttggggcttg gaaggttcct 5880

gtcgaagaaa aagtcaggaa agataacaat tgcgcacttg cagtggacaa cgcttccctg 5940

tcttctatgc tataggtgga cagcattttt ctaggtataa ttaatttgac cttcaaacat 6000

atgtatacta accaacgcgg ttttgattcc atcaaatgtt ttggactctc tctgctgaac 6060

tgtcaaagtt acttcatggg gcaaaatgtc aaattttctg gaaccttccg tagtatattt 6120

tggaaatgag tgtttattgt gtcattggaa ataccgttca tgtgtctgtg acagaatgtg 6180

tcactagaaa gctgaattgg tgttgtcctt gtcaaaaagg cactaaacac gagtctgaaa 6240

attaggcctg ttcttggtaa gggaaggaat ctgagcatca atgctgatag gaatagactc 6300

tgtctgtcaa tattgttaac ttgtttatag ggcttcgagt tttcaacttt tgaggcagat 6360

aagtaggata cctcttttga tcatgatata taacatattc ttatatacct caagccttgc 6420

actgttaagt taatgtggca tcctttctag agatcatgac ctcaagttgc atatggatgc 6480

caataatatc gacaccaagt gaacatcagt gtctgtggaa tatgccgaaa gcagccaacg 6540

tgccattact gaattttcat atgattatta tattctgttt agatttattt acgtcggaac 6600

acagtgagat ggtaacgtaa tgaatcaaaa taggctataa acatgcaatt caacatatca 6660

ttatcatgcc caagtgtttt gtcattctat ctttattcgt ccaagaagga caagcctggt 6720

gcattgttga gggaaccagt tcttctgcag tacttctagg gaggtaaaaa ttcaacaccg 6780

ttggatgcag atctatcgaa cccagggact ttgtgcttcc agtgaaaagt tatatggacc 6840

cataggccag aggatgtgag agttttacct ctctggaagt tatatgcgct agcattagtg 6900

tggtcatcaa tgggatcaaa gatgagctcc acctttggtg tagagctgga gctaggggac 6960

tctagcatcc tggcgctcca atcttccatc cagtgaactc tgtttttggg gtctagtagg 7020

tcaagggtcc agttattttt tctttctgct gtaaagtctc tagttaaggt gtgagttttg 7080

tatggtgttt tttcgaggtt tccccaaacc tcaccttttt tccttcttaa tataatgata 7140

tgcagctttc ctgcgtattc gagaaaagaa agttttatct ctctggaagt taactgcaga 7200

ggaacttgtt acattgttga gagttgtctc accgagtcac caggtcgctg gttcaaagca 7260

gtctctccac atttatgtgg aaggcttgcc tcggtttatc ccttcccaag actctacttg 7320

tgggagactc tggcattggg tctgtcctat gccgttgaag cgctaggttc gtttatccct 7380

tccctatacc cacttgtcag agcctccaac actgagtctg ccctaagctt ccaagttcca 7440

acactgggtc tgccctaggc cgtcgaagcg ttatatgatt gccatgtact gttatgcttt 7500

gttgccttca catatttccc gttcgaaatc atctccttgt tgccttcaca tattgccttg 7560

ttgctttcac atattttctg ttccacgtca tacttagaag ttagaacacg tgatttatgc 7620

caattaagat tattatttta tataacagat gacccgccct gttgctgtgg aggatgtgaa 7680

aagagaagtg aagattctta aagcacttaa aggacatcag aatattgttc acttctacaa 7740

tgcatttgag gatgattcat acgtgtacat tgtgatggag taagtaggcc catacacctg 7800

ttcctgctaa tagagcatat cgattttgct atgacttttt tccctaaagt tttaacatga 7860

acaatatcta tcctgtttac agaatcctag acactaaaat gtcatttcta attatcaatt 7920

attctatagc taaaccagat gcaatcctga tttatttttc ttaacgtatg gatatattgg 7980

acttttcttt caaacctgca ttttgaattt gattacaggg aactataaca ctaattcaga 8040

actctatcat gtttaacatt tttcttgcat tgttctatgt ttgtcaactt gacgcacttc 8100

ttagataata taacatcatc ttccacagtc accattagtt aggaccttgg accttcatgg 8160

ttccgaaatt tagctaagaa tggtatacat ggtcatgtga tttcaaatag atgttcctat 8220

atgccagaac caactcataa gtcataagtt ttaccttgtg tttttgcagg ctatgtgagg 8280

gcggtgaact attagatcgg attttggcaa agtaagtaga taagatcccc atctctttgt 8340

ttcccgtacc tcattcttcg ccattaaatt tatagatttt tgtgctgtaa aatcagattg 8400

ctttatgttg tttgtctgct ttgtttgatt tctagttgct cgttcaagat cctttactta 8460

atggtgtgcg tgttttgaca gaaagaatag ccgctatagt gagaaagatg ctgcagtggt 8520

agtccgccaa atgctcaaag tagctgctga atgccatctg cgtgggttag ttcaccgaga 8580

tatgaagcct gaggtagaaa tcaaatactt caatctcttt gcacacagta agcatttggt 8640

gatatttcac tacttcctca ggtcatgtaa gactgtacct attttccttc ccagaacttc 8700

cttttcaaat cgaacaagga ggattcacca ctaaaggcga cagattttgg tttgtcagat 8760

ttcattaagc caggtatcta cttggggcca tctgaatctg tcgggaatct gataggggca 8820

agtctgcagt ttagctgacc attttgttgt ctaatgcatg ctttagggaa gaagttccat 8880

gacattgttg gaagtgctta ctatgtcgca ccagaagtac taaaacgacg gtctggtcct 8940

gagtcagatg tttggagcat aggagtcata acctacattt tgctctgtgg gaggcgccct 9000

ttttgggata agaccgaaga cggtatattc aaggaggtaa gtggatggat tttgcatacc 9060

atgtgcttac atgtaaaata tgcttggtta gagtgctgta ccagggatca gcgttttcag 9120

cgtgctgata ctgttttgta caatgtgttt ctactttcta cgtcatatag cagtgtttct 9180

ttgttaacta tttcagtgtc aaactatttg tcgtgtcaca actcagcagt ataattttac 9240

tattttgaac actgtaaacc tgcctggtca ggttatcctt cagtaatttc tctactagct 9300

accagaaacc cactttatgc aggtgttcag tttaataaca cccaccatct ttcagatttc 9360

taatgttcag tgttagacag acttcattaa gatgcacctt aagatgattg taagtagtaa 9420

aagtgctttg cacttttgtt aacttttgag tctgaagatg acttgtggta cctatgacct 9480

caagaaacca aggcattgcc attggaatag ctaattcgaa tgagcttcag atatggctat 9540

ctgttttagt tttggacatc tgactcaact ttataggata atactatatt agcaatcttt 9600

gaggtcattg tctcagccaa aataagttgc ggtctctttt ttactgtcct aagcagcaat 9660

atggtttcca ttttcattat accagcaact tccacctttt tcttgctatt taaatatctt 9720

tatgcatttt atcagcaagg acatgatacg atcgtatatg tgatattcta catcttttca 9780

cttctcataa ttaggttcta aggaacaagc ctgattttcg taagaggcct tggtcaagca 9840

tcagcccagg tgctaaagat tttgttaaaa ggttactagt gaagaatcca agggccaggc 9900

taacagctgc tcaagctctc tgtaagtttt ggtatttttc attaatttac tagcctagtc 9960

atgatgatca gattcacctt ctctatgtga gaacagagaa cacatataca tctggcagta 10020

tgcctttcaa tcagttatga caatgtaaat atgcaaagac cgatgttttt tctatcctgc 10080

accattttag aacattaatg gggaaaaacc acaatatatt aggaaaaatg tttaattatg 10140

tcctggtcac ttgaaatgaa catataccac tgaggttttc tagttctcat gcgttcttat 10200

aatgatctaa taagtcagtg gaggtttgct gcccaccacc cctacatttg tattgtgaat 10260

tactatcatc tttactgatc ctgattgttc ttgatatgtt aagcacatcc gtgggtaaga 10320

gaaggagggg aagcatccga tatccccgtc gacatatctg tgttatcaaa catgcgtcag 10380

tttgtcaagt acagccgttt caagcaattc gcgcttcggg taattacagt gattacaaaa 10440

aacaacactg catcgtttat tttttcctca caatatttcc tcgtggcatg gtcaggctct 10500

ggcgagcacc cttaacgagg aagagctatc agatctgaag gatcagtttg atgcaattga 10560

tatcgataaa agtggatcga ttagtatcga ggaaatgcgt catgtaggtt ctgttagtgt 10620

ttgctgatga aaatgcctta gatcctgaac tactctgcgg tgctgattaa tctgtgcatg 10680

tttcggtagg cccttgcaaa ggatcttccc tggagattga agggtccccg tgtgctggag 10740

attattcaag cagtaagttt gagccttctt ctggatccag ccctttcttt gttacccccc 10800

ttgtttccaa gaaaatagct ggccttgttc tgagggtata accaaaactg catcttattt 10860

tgtggtagat tgacagcaac actgatgggc tcgtggactt caaggagttt gttgcggcaa 10920

ctctccatat ccaccagatg gcggagctcg actcagaaag gtggggcata cgctgccaag 10980

ctgctttcag taagtttgat cttgacggtg atggatatat cacgccggag gaactcagaa 11040

tggtaatttt ctactcctgt cttgtttcca tgttgcttca ccaacgaatg cacagttcac 11100

ataaccctta ttatcatcac tgcttcccat gaataactag ctggctcgac catcatgaga 11160

ttcagtactt gcgccctgtg cacttggttt tggtcccgct tgttagaatg aagtaattta 11220

tcaatggaag cgctgtaata ttttaatcag cgtttagatt tgataaagat aaaacatgtt 11280

cattgtttgt gccaagaaat ccacttacac agatactgag agttgcaccg tagataacgc 11340

taatcggcag tatcctaatc gagattttct ttcaaggtgc agcaccctgg gttgaaggga 11400

tctatcgagc cgctgctgga ggaggccgac atcgacaaag acggcaagat aagcctgtcc 11460

gagttccgca agctcctacg gacagcgagc atgagcaacg tacccagccc aagggggccc 11520

ccaaaccctc aggctctgtg aattccggct cggccactag ggaggagcaa gcttaggaag 11580

ttgccataca atagccatgt gttctttggg ttcttcagag tgccatgtga tgtttctggt 11640

ttttagcatc caggttatgt gtgcagtgca gccccgagtg agtttcgaag taaatattca 11700

gtgctttctt tttctttccg gaagagtgag aggtggaggt caaaatggta ggcaagactc 11760

gccttcttct ttcctttaca ctgtacagtg atactgaaat atgtacgatt tttattataa 11820

ctgttcgtcg caataaagtt atttggagaa gtgaggattt tattgtcctg gtgaacctgt 11880

acgttttttc cccaaacgga tcacggtcac ggcccccaag tttcagcata aaaagtttat 11940

gacagatttc tggtttggtt cgctgcctca ttcatgctgt tcgtttggga gtaaaactag 12000

ttgttgcaac tgtaatctat agagacaatt agataaaata ctgagattaa agctaaatga 12060

acagactgcc acaatttatt atgtttaagg ttagatattt aatcgattta ggcgtgtgtt 12120

tggtttatag ctataattgt gatatttttt ttttctgttg tgtagatttt acttgtcaac 12180

aattgtgatt tttttcttct gttgtgtaga ttttacttgt caacaattta ttatgtttaa 12240

ggttagacat ttaatcgatt tagacgtgtg tttggtttat agctataatt gtgatatttt 12300

ttttctgttg tgtagatttc attgtaaaag tgtgatagtt ttttaggttt ttctaaatgt 12360

agtgaagaat ttgagcgcag acaggtgtgg cagaaaactg tgtaccacca aacagcccct 12420

tcatttgcag ttccattgcc gcgacaagca attgcggttg atgattgagg atccggtggc 12480

agtacagcag actgcccgtt cggaaaagcg cgcgggggct tctccctagt cgcttggcag 12540

gctgaatgcc ggcaccgcgg ttggcgttaa acccggccgc cgtgctccac gcggcgctcc 12600

tcagggcctc ctctgccggt tgccgcctgc ctccccgcat ctccttcaac tcgctgctgg 12660

cggccgccgc gtcctccgcg gacacgcgca cccgcgctct cgcgctcccg gctctcgcgc 12720

tcgcccacgc ctccggccgc gtgtccctcg actcgtacgc cctctgctcc gcgctccgct 12780

ccgcgccctc cgccgcgggg acgctgcacg cgctggccgc caagtccggc tggctcggca 12840

gcgtcttcgt gtcctgcgcg ctcgccgctt cctacggcgg gtccggccgg tgcctggacg 12900

cccggagcct gttcgacgaa agtcccgcca ggaacggcgt cttcgggaac gccgtcctcg 12960

ccgcttacgt gggcgcggcc gagtgggctc ccgtgctgag gttcgccagg aggttctcgg 13020

aactgcggct gcaggttgac tggtacacga tgacggctgt ggcgcgggcg tgtggcgagg 13080

tggccaacgc tgatctcggc gtccaggcgc atgggcatgc ggtcaggagg ctgggaggtg 13140

tagaggtgga cgtgttcttg gtcagcgcgt tcgtggacat gtacgccaag tgcgggctta 13200

tcagccaagc ggagcgtgtg ttccgccttg cgcaacagga gaccggtggc agaggtgacg 13260

ttgtgctgtg gacggccatg ttgaacgcct atgcgcggca tggacagtgc aaggaggtta 13320

tccggcagta tgacctgatg ctggcctctg gtgtctatcc ggatgaattg gccatgttag 13380

ctgtactctc agcttgccag cacgccgggg aggtggtcaa ggggctcaac tactttgaat 13440

ccatgcatgc agattacggg ctggtgccca caccggagca ctacggttgt gtggtcaaca 13500

tgctgtgccg ggcaggggaa gtgaccaagg cgtgggagat tgccaccaag gacggctgtg 13560

atc 13563

<210> 13
<211> 2331
<212> DNA
<213> Artificial sequence

<400> 13
atgggcgctt gcttctcctc cgcctctgcc gcccccgccg gcgccgccgt cgacgagcgc 60

cgcccgtcca aggagggcga cggcaagaag aggcgccgcg ccgccggggc atcgccggat 120

gccgcggcgc ccgtgcgcgt ggagttcggc tacgagaggg acttcgaggc gcgctacgag 180

gtcggccgcc tgctcggcca cggccagttc ggctacacct tcgccgccac cgaccgcggc 240

tctggggacc gcgttgccgt caagcgcatc gacaaggcca agatgacccg ccctgttgct 300

gtggaggatg tgaaaagaga agtgaagatt cttaaagcac ttaaaggaca tcagaatatt 360

gttcacttct acaatgcatt tgaggatgat tcatacgtgt acattgtgat ggagctatgt 420

gagggcggtg aactattaga tcggattttg gcaaaaaaga atagccgcta tagtgagaaa 480

gatgctgcag tggtagtccg ccaaatgctc aaagtagctg ctgaatgcca tctgcgtggg 540

ttagttcacc gagatatgaa gcctgagaac ttccttttca aatcgaacaa ggaggattca 600

ccactaaagg cgacagattt tggtttgtca gatttcatta agccagggaa gaagttccat 660

gacattgttg gaagtgctta ctatgtcgca ccagaagtac taaaacgacg gtctggtcct 720

gagtcagatg tttggagcat aggagtcata acctacattt tgctctgtgg gaggcgccct 780

ttttgggata agaccgaaga cggtatattc aaggaggttc taaggaacaa gcctgatttt 840

cgtaagaggc cttggtcaag catcagccca ggtgctaaag attttgttaa aaggttacta 900

gtgaagaatc caagggccag gctaacagct gctcaagctc tctcacatcc gtgggtaaga 960

gaaggagggg aagcatccga tatccccgtc gacatatctg tgttatcaaa catgcgtcag 1020

tttgtcaagt acagccgttt caagcaattc gcgcttcggg ctctggcgag cacccttaac 1080

gaggaagagc tatcagatct gaaggatcag tttgatgcaa ttgatatcga taaaagtgga 1140

tcgattagta tcgaggaaat gcgtcatgcc cttgcaaagg atcttccctg gagattgaag 1200

ggtccccgtg tgctggagat tattcaagca attgacagca acactgatgg gctcgtggac 1260

ttcaaggagt ttgttgcggc aactctccat atccaccaga tggcggagct cgactcagaa 1320

aggtggggca tacgctgcca agctgctttc agtaagtttg atcttgacgg tgatggatat 1380

atcacgccgg aggaactcag aatgcaccct gggttgaagg gatctatcga gccgctgctg 1440

gaggaggccg acatcgacaa agacggcaag ataagcctgt ccgagttccg caagctccta 1500

cggacagcga gcatgagcaa cgtacccagc ccaagggggc ccccaaaccc tcaggctctg 1560

gatccggctg ctgccgctgc cgctgcggca gcggccggac cggtcgccac catggtgagc 1620

aagggcgagg agctgttcac cggggtggtg cccatcctgg tcgagctgga cggcgacgta 1680

aacggccaca agttcagcgt gtccggcgag ggcgagggcg atgccaccta cggcaagctg 1740

accctgaagt tcatctgcac caccggcaag ctgcccgtgc cctggcccac cctcgtgacc 1800

accctgacct acggcgtgca gtgcttcagc cgctaccccg accacatgaa gcagcacgac 1860

ttcttcaagt ccgccatgcc cgaaggctac gtccaggagc gcaccatctt cttcaaggac 1920

gacggcaact acaagacccg cgccgaggtg aagttcgagg gcgacaccct ggtgaaccgc 1980

atcgagctga agggcatcga cttcaaggag gacggcaaca tcctggggca caagctggag 2040

tacaactaca acagccacaa cgtctatatc atggccgaca agcagaagaa cggcatcaag 2100

gtgaacttca agatccgcca caacatcgag gacggcagcg tgcagctcgc cgaccactac 2160

cagcagaaca cccccatcgg cgacggcccc gtgctgctgc ccgacaacca ctacctgagc 2220

acccagtccg ccctgagcaa agaccccaac gagaagcgcg atcacatggt cctgctggag 2280

ttcgtgaccg ccgccgggat cactctcggc atggacgagc tgtacaagta a 2331

<210> 14
<211> 2337
<212> DNA
<213> Artificial sequence

<400> 14
   atgggcgctt gcttctcctc cgcctctgcc gcccccgccg gcgccgccgt cgacgagcgc 60
   cgcccgtcca aggagggcga cggcaagaag aggcgccgcg ccgccggggc atcgccggat 120
   gccgcggcgc ccgtgcgcgt ggagttcggc tacgagaggg acttcgaggc gcgctacgag 180
   gtcggccgcc tgctcggcca cggccagttc ggctacacct tcgccgccac cgaccgcggc 240
   tctggggacc gcgttgccgt caagcgcatc gacaaggcca agatgacccg ccctgttgct 300
   gtggaggatg tgaaaagaga agtgaagatt cttaaagcac ttaaaggaca tcagaatatt 360
   gttcacttct acaatgcatt tgaggatgat tcatacgtgt acattgtgat ggagctatgt 420
   gagggcggtg aactattaga tcggattttg gcaaaaaaga atagccgcta tagtgagaaa 480
   gatgctgcag tggtagtccg ccaaatgctc aaagtagctg ctgaatgcca tctgcgtggg 540
   ttagttcacc gagatatgaa gcctgagaac ttccttttca aatcgaacaa ggaggattca 600
   ccactaaagg cgacagattt tggtttgtca gatttcatta agccagggaa gaagttccat 660
   gacattgttg gaagtgctta ctatgtcgca ccagaagtac taaaacgacg gtctggtcct 720
   gagtcagatg tttggagcat aggagtcata acctacattt tgctctgtgg gaggcgccct 780
   ttttgggata agaccgaaga cggtatattc aaggaggttc taaggaacaa gcctgatttt 840
   cgtaagaggc cttggtcaag catcagccca ggtgctaaag attttgttaa aaggttacta 900
   gtgaagaatc caagggccag gctaacagct gctcaagctc tctcacatcc gtgggtaaga 960
   gaaggagggg aagcatccga tatccccgtc gacatatctg tgttatcaaa catgcgtcag 1020
   tttgtcaagt acagccgttt caagcaattc gcgcttcggg ctctggcgag cacccttaac 1080
   gaggaagagc tatcagatct gaaggatcag tttgatgcaa ttgatatcga taaaagtgga 1140
   tcgattagta tcgaggaaat gcgtcatgcc cttgcaaagg atcttccctg gagattgaag 1200
   ggtccccgtg tgctggagat tattcaagca attgacagca acactgatgg gctcgtggac 1260
   ttcaaggagt ttgttgcggc aactctccat atccaccaga tggcggagct cgactcagaa 1320
   aggtggggca tacgctgcca agctgctttc agtaagtttg atcttgacgg tgatggatat 1380
   atcacgccgg aggaactcag aatggtgcag caccctgggt tgaagggatc tatcgagccg 1440
   ctgctggagg aggccgacat cgacaaagac ggcaagataa gcctgtccga gttccgcaag 1500
   ctcctacgga cagcgagcat gagcaacgta cccagcccaa gggggccccc aaaccctcag 1560
   gctctggatc cggctgctgc cgctgccgct gcggcagcgg ccggaccggt cgccaccatg 1620
   gtgagcaagg gcgaggagct gttcaccggg gtggtgccca tcctggtcga gctggacggc 1680
   gacgtaaacg gccacaagtt cagcgtgtcc ggcgagggcg agggcgatgc cacctacggc 1740
   aagctgaccc tgaagttcat ctgcaccacc ggcaagctgc ccgtgccctg gcccaccctc 1800
   gtgaccaccc tgacctacgg cgtgcagtgc ttcagccgct accccgacca catgaagcag 1860
   cacgacttct tcaagtccgc catgcccgaa ggctacgtcc aggagcgcac catcttcttc 1920
   aaggacgacg gcaactacaa gacccgcgcc gaggtgaagt tcgagggcga caccctggtg 1980
   aaccgcatcg agctgaaggg catcgacttc aaggaggacg gcaacatcct ggggcacaag 2040
   ctggagtaca actacaacag ccacaacgtc tatatcatgg ccgacaagca gaagaacggc 2100
   atcaaggtga acttcaagat ccgccacaac atcgaggacg gcagcgtgca gctcgccgac 2160
   cactaccagc agaacacccc catcggcgac ggccccgtgc tgctgcccga caaccactac 2220
   ctgagcaccc agtccgccct gagcaaagac cccaacgaga agcgcgatca catggtcctg 2280
   ctggagttcg tgaccgccgc cgggatcact ctcggcatgg acgagctgta caagtaa 2337

<210> 15
<211> 239
<212> PRT
<213> Artificial sequence

<400> 15

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  16
<211>  953
<212>  DNA
<213>  Artificial sequence

<400>  16
ggtctctggc gacaagcctg attttcgtaa ggttttagag ctagaaatag caagttaaaa      60

taaggctagt ccgttatcaa cttgaaaaag tggcaccgag tcggtgcttt tttttttcgt     120

tttgcattga gttttctccg tcgcatgttt gcagttttat tttccgtttt gcattgaaat     180

ttctccgtct catgtttgca gcgtgttcaa aaagtacgca gctgtatttc acttatttac     240

ggcgccacat tttcatgccg tttgtgccaa ctatcccgag ctagtgaata cagcttggct     300

tcacacaaca ctggtgaccc gctgacctgc tcgtacctcg taccgtcgta cggcacagca     360

tttggaatta aagggtgtga tcgatactgc ttgctgctca tgaatccaaa ccacacggag     420

ttcaaattcc cacagattaa ggctcgtccg tcgcacaagg taatgtgtga atattatatc     480

tgtcgtgcaa aattgcctgg cctgcacaat tgctgttata gttggcggca gggagagttt     540

taacattgac tagcgtgctg ataatttgtg agaaataata attgacaagt agatactgac     600

atttgagaag agcttctgaa ctgttattag taacaaaaat ggaaagctga tgcacggaaa 660

aaggaaagaa aaagccatac ttttttttag gtaggaaaag aaaaagccat acgagactga 720

tgtctctcag atgggccggg atctgtctat ctagcaggca gcagcccacc aacctcacgg 780

gccagcaatt acgagtcctt ctaaaagctc ccgccgaggg gcgctggcgc tgctgtgcag 840

cagcacgtct aacattagtc ccacctcgcc agtttacagg gagcagaacc agcttataag 900

cggaggcgcg gcaccaagaa gctgatgggc tcgtggactt cagtttagag acc 953

<210> 17
<211> 20
<212> RNA
<213> Artificial sequence

<400> 17
acaagccuga uuuucguaag 20

<210> 18
<211> 20
<212> RNA
<213> Artificial sequence

<400> 18
ugaugggcuc guggacuuca 20

<210> 19
<211> 17
<212> PRT
<213> Artificial sequence

<400> 19

Asp Pro Ala Ala Ala Ala Ala Ala Ala Ala Ala Ala Gly Pro Val Ala
1               5                   10                  15

Thr